# EUROPEAN PATENT SPECIFICATION

(11) **EP 3 512 403 B1**
(45) Date of publication and mention of the grant of the patent: **14.12.2022**
(21) Application number: 17849927.3
(22) Date of filing: 15.09.2017
(51) Int. Cl.: A61B 1/04, A61B 1/06, A61B 1/24, A61B 1/227, A61B 1/233, A61B 1/00, A61B 1/05

(54) **ORIFICE INSPECTION SYSTEM**
SYSTEM ZUR INSPEKTION VON ÖFFNUNGEN
SYSTÈME D'INSPECTION D'ORIFICE

(30) Priority: 16.09.2016 AU 2016903740; 16.09.2016 AU 2016903742
(43) Date of publication of application: 24.07.2019
(73) Proprietor: Throat Scope Pty Ltd, Brisbane, QLD, 4000 (AU)
(72) Inventor: HOLLAND, Jennifer Louise, Brisbane, QLD, 4000 (AU)
(74) Representative: London IP Ltd
(86) International application number: PCT/AU2017/051004
(87) International publication number: WO 2018/049479

(56) References cited:
- EP-A1- 2 789 290
- WO-A1-2005/016135
- WO-A1-2012/154578
- WO-A1-2016/043511
- CN-U- 205 548 507
- KR-A- 20160 044 987
- KR-A- 20160 044 987
- KR-B1- 101 596 417
- US-A1- 2013 209 954

## Description

### TECHNICAL FIELD

The present invention relates to a system for inspecting a patient's orifice, especially the patient's throat, ear or nose, to components of the system, and to methods of inspecting a patient's orifice.

### BACKGROUND ART

It will be clearly understood that, if a prior art publication is referred to herein, this reference does not constitute an admission that the publication forms part of the common general knowledge in the art in Australia or in any other country.

The discussion below relates to inspection systems for ears and throats. However, for the avoidance of doubt, the specification is not limited to systems for inspecting ears and throats.

Typically a patient suffering from discomfort in their ear or throat has the choice of waiting to see if the discomfort goes away, or visiting a medical professional. In some cases, neither option is ideal. For example, if a patient waits to see if the discomfort goes away, the discomfort may become worse over time or may be unchanged. In the meantime, the patient has had to suffer through prolonged discomfort. Alternatively, it can be difficult for the patient to visit a medical professional due to cost or availability. For example, the patient may have other commitments making it difficult to find time to see the medical professional, the medical professional may be heavily booked preventing an immediate visit, or the patient may need to travel some distance to see the medical professional.

KR20160044987 discloses an endoscope inspection device using a mobile terminal. US2013209954A1 discloses a method, system and apparatus for imaging an oral cavity of a subject. WO2012154578A1 discloses a personal electronic device endoscope image and diagnosis capture system. WO2016043511A1 discloses an endoscope device for a portable terminal. WO2005016135A1 discloses an adapter for coupling an optical system, such as an endoscope, to a videophone. KR101596417B1 discloses a mobile endoscope apparatus which comprises: a probe module inserted into the human body; a light source module for supplying light into the human body through the probe module; and, a mobile terminal which comprises: an image module for using the light to shoot the image of the inside of the human body, toward which the probe module heads, and to convert the shot image into an image signal; and, a display module for receiving the image signal to display.

### SUMMARY OF INVENTION

The invention for which protection is granted is defined in independent claim 1 with dependent claims 2 - 11 defining preferred embodiments thereof. Any other aspects or embodiments described in the description not falling within the scope of these claims do not form part of the protected subject-matter. The present invention is directed to an orifice inspection system which may at least partially overcome at least one of the abovementioned disadvantages or provide the consumer with a useful or commercial choice. In one aspect, the present invention is directed to an orifice inspection system, which may allow a user to perform improved self-assessment of a potential condition affecting a patient's orifice, or to allow a medical professional to perform a diagnosis of a condition affecting a patient's orifice remote to the patient.

With the foregoing in view, the present invention in one form, resides broadly in an orifice inspection system . An orifice inspection system according to the claimed invention includes an orifice inspection device for illuminating a patient's orifice, the orifice inspection device including a handle with a light source, a power source and a disposable light transmissive structure formed of a light transmissive material releasably connectable to the handle, the handle including a handle body and a coupling portion extending therefrom, the light source being located within the coupling portion of the handle and the coupling portion including a switch in communication with the light source and the power source, and the disposable light transmissive structure includes a cavity to receive the coupling portion of the handle and activate the switch such that the light source is directed therealong.

The orifice inspection system according to the claimed invention further includes a smart phone including a screen and an image capture device for capturing a photograph of the patient's orifice as illuminated by the orifice inspection device; and a coupler arranged to attach with the smart phone so as to releasably couple the handle body of the orifice inspection device to the smart phone thereby positioning the disposable light transmissive structure relative to the image capture device.

In the orifice inspection system according to the claimed invention the orifice inspection device is operable in a first condition disconnected from the smart phone in which a user may clasp the handle body to illuminate the patient's orifice, and a second condition in which the handle body is coupled to the smart phone so as to be immediately adjacent the smart phone with the disposable light transmissive structure laterally spaced apart from the image capture device so that the image capture device is unobstructed thereby allowing the user to capture the photograph of the patient's orifice surrounding the disposable light transmissive structure whilst holding the smart phone.

In a first aspect, the present invention relates to an orifice inspection system including:
- An image capture device for capturing a photograph of a patient's orifice; and
- A graphical user interface including an orifice positioning guide for positioning a patient's orifice in the photograph captured by the image capturing device.

Advantageously, a graphical user interface including an orifice positioning guide for positioning a patient's orifice in the photograph captured by the image capturing device may allow a user to capture improved photographs of the patient's orifice. This may allow the user or a medical professional inspecting the photograph to better evaluate the condition affecting the patient's orifice.

In one embodiment of the first aspect, the system further includes a data interface for transmitting the photograph to the patient's medical service provider. In a further embodiment of the first aspect, the graphical user interface is configured to display the captured photograph and at least one photograph of an orifice having a known condition for comparison, to assist in identifying a condition affecting the patient's orifice. In yet another embodiment of the first aspect, the system further includes an orifice inspection device for illuminating a patient's orifice.

In a second aspect, the present invention relates to an orifice inspection system including:
- An image capture device for capturing a photograph of a patient's orifice; and
- A data interface for transmitting the photograph to the patient's medical service provider.

Advantageously, a data interface for transmitting the photograph to the patient's medical service provider may allow a user to easily transmit a photograph to the medical service provider. In turn, this may allow the medical service provider to remotely diagnose a condition affecting the patient's orifice, or may allow the medical service provider to assess whether or not they need to see the patient in person.

In one embodiment of the second aspect, the system further includes a graphical user interface including an orifice positioning guide for positioning a patient's orifice in the photograph captured by the image capturing device. In a further embodiment of the second aspect, the system further includes a graphical user interface configured to display the captured photograph and at least one photograph of an orifice having a known condition for comparison, to assist in identifying a condition affecting the patient's orifice. In yet another embodiment of the second aspect, the system further includes an orifice inspection device for illuminating a patient's orifice.

In a third aspect, the present invention relates to an orifice inspection system including:
- An image capture device for capturing a photograph of a patient's orifice; and
- A graphical user interface configured to display the captured photograph and at least one photograph of an orifice having a known condition for comparison, to assist in identifying a condition affecting the patient's orifice.

Advantageously, a graphical user interface configured to display the captured photograph and at least one photograph of an orifice having a known condition for comparison, to assist in identifying a condition affecting the patient's orifice may allow a user to better self-evaluate a condition possibly affecting the patient's orifice. In turn, this may allow the user to make a more informed decision as to whether or not it is necessary to visit a medical service provider to obtain medical advice.

In one embodiment of the third aspect, the system further includes a data interface for transmitting the photograph to the patient's medical service provider. In a further embodiment of the third aspect, the graphical user interface further includes an orifice positioning guide for positioning a patient's orifice in the photograph captured by the image capturing device. In yet another embodiment of the third aspect, the system further includes an orifice inspection device for illuminating a patient's orifice.

In a fourth aspect, the present invention relates to an orifice inspection system, including:
- An orifice inspection device for illuminating a patient's orifice; and
- An image capture device for capturing a photograph of the patient's orifice as illuminated by the device.

Advantageously, an orifice inspection device for illuminating a patient's orifice may allow a user to provide improved illumination of a patient's orifice which in turn allows for a clearer photograph to be captured by the image capture device. The orifice inspection device may be an illuminated orifice inspection device.

In one embodiment of the fourth aspect, there is provided an orifice inspection system, including:
- An orifice inspection device for illuminating a patient's orifice; and
- An image capture device for capturing a photograph of the patient's orifice as illuminated by the orifice inspection device;
wherein the orifice inspection device is mountable relative to the image capture device, or wherein the image capture device is mounted on the orifice inspection device. Advantageously, the system of this embodiment may allow for a user to capture a photograph of the patient's orifice with one hand. Advantageously, the system of this embodiment may allow for a user to more easily capture a photograph of their own orifice.

In one embodiment of the fourth aspect, the system further includes a graphical user interface including an orifice positioning guide for positioning a patient's orifice in the photograph captured by the image capturing device. In another embodiment of the fourth aspect, the system further includes a data interface for transmitting the photograph to the patient's medical service provider. In a further embodiment of the fourth aspect, the system further includes a graphical user interface configured to display the captured photograph and at least one photograph of an orifice having a known condition for comparison, to assist in identifying a condition affecting the patient's orifice.

Features of the systems of the first to fourth aspects of the present invention may be as further described below.

As used herein, the term "orifice" includes any cavity or opening to a passage in the patient's body. In one embodiment, the orifice inspection device includes a light source, and the orifice inspection device is for inspecting an oral cavity, an outer ear or a nostril. In one embodiment, the orifice includes an oral cavity, at least one nostril (or nasal passage or nares), or an outer ear. The oral cavity may include at least one of the group consisting of: at least one cheek (or buccal region), tongue (or lingual region), beneath the tongue (or sub-lingual region), at least one tonsil (including palatine tonsils, nasopharyngeal tonsil (or adenoid) or lingual tonsils), uvula, pharynx and palate (including soft palate (or velum) or hard palate). The oral cavity may especially include at least one of (or all of) the group consisting of: palatine tonsils, adenoid, uvula and pharynx. The patient may be an animal, especially a human. However, the orifice inspection system may also be for veterinary use.

The outer ear may include one or more of the tragus, the anti tragus, the intertragic notch, the concha cavum, the eardrum (the tympanic membrane) and the outer ear canal (the external acoustic meatus). The outer ear may especially include the eardrum and/or the outer ear canal (which may be all of the outer ear canal or at least a portion of the outer ear canal, as inspected without moving an otoscope in the ear).

The orifice inspection system of the first to fourth aspects of the present invention includes an image capture device for capturing a photograph of a patient's orifice. The photograph may be an image captured in the visible spectrum. The image capture device may be any device capable of taking a photograph. According to the disclosure, the image capture device is a camera, camera module, webcam or film.

The orifice inspection system includes a smart phone including a screen and an image capturing device.

The smart phone may also include the graphical user interface. The user device may also include the data interface. In one embodiment, the user device is a smartphone including a camera, a graphical user interface and a data interface.

In another configuration, not falling within the scope of the claims, the image capture device may be a webcam, especially a webcam connectable to a computer. In this embodiment, the user device may be a computer. The computer may include a data interface, and a graphical user interface.

In a further configuration, not falling within the scope of the claims, the image capture device may be a film. The film may be mounted on the orifice inspection device, for example on an orifice inserter (as discussed below).

In one embodiment, the orifice inspection system may disable a flash associated with an image capture device. Sufficient light for the photograph may be provided by the orifice inspection device.

The graphical user interface may include the orifice positioning guide. The orifice positioning guide may be instructions to guide a user to correctly position the patient's orifice in the photograph to be captured by the image capturing device. In another embodiment, the graphical user interface includes an image to be captured by the image capturing device, and the orifice positioning guide is a graphical guide (or diagrammatic guide) overlaid on said image. In this way, when the orifice is aligned with the orifice positioning guide the user can capture the photograph. In another embodiment, the graphical user interface includes an image to be captured by the image capturing device.

In one embodiment, the orifice positioning guide is a line overlaid on an image to be captured by the image capturing device. In another embodiment, the orifice positioning guide is a semi-transparent image of all or a portion of a correctly positioned orifice, which is overlaid on an image to be captured by the image capturing device. For example, if the orifice is the oral cavity, the orifice positioning guide may be a line illustrating the opening to the pharynx. In another example, if the orifice is the oral cavity, the orifice positioning guide may be a line illustrating the position of one or more of the palatine tonsils and the uvula (especially all of the palatine tonsils and the uvula). In a further example, if the orifice is the outer ear, the orifice positioning guide may be a line illustrating the position of the tympanic membrane.

In one embodiment, the orifice inspection system is configured to detect the presence of the orifice inspection device, and to automatically adjust the image magnification of the image capture device based on the location of the orifice inspection device. In one embodiment, the orifice inspection system is configured to detect the presence of the orifice inspection device based on user input. For example, a graphical user interface may include a menu querying if an orifice inspection device is mounted relative to or mounted to an image capture device or a user device including an image capture device. If the user confirms that the orifice inspection device is mounted relative to or mounted to an image capture device or a user device including an image capture device, then the image magnification of the image capture device may be automatically adjusted. The image magnification may be automatically adjusted in any suitable way. In one embodiment, the orifice inspection system may detect or locate the position of the orifice inserter and automatically adjust the image magnification so the image includes a defined portion (or none) of the orifice inserter. In another embodiment, the orifice inspection system may set the image magnification based on a defined distance between the image capture device and the orifice inserter. The orifice inspection system may include a graphical user interface which is configured to manually adjust the image magnification. The orifice inspection system may, for example, adjust the focal length of a lens in the image capture device, or digitally zoom (or enlarge) the image to be captured by the image capture device, with or without user input.

In one embodiment, the graphical user interface may include a menu querying what type of orifice inspection device is present. Selection of the type of orifice inspection device may accordingly change the graphical user interface. For example, if the orifice inspection device is a tongue depressor, then the database may include images of an oral cavity. Alternatively, if the orifice inspection device is an otoscope, then the database may include images of an outer ear. In one embodiment, the user registers the orifice inspection device and/or the orifice inserter, to confirm the type of orifice inspection device. In an exemplary embodiment, the orifice inspection system may automatically register the orifice inspection device and/or the orifice inserter as the orifice inspection device and/or the orifice inserter is placed in proximity to the image capture device. For example, the orifice inspect device and/or the orifice inserter may include a radio frequency identification tag.

The orifice inspection system may include a graphical user interface for modifying a captured image (or photograph). For example, the graphical user interface may allow a user to highlight (or circle) a portion of an image. Advantageously, in this way a user may be able to highlight regions of the photograph of particular concern to a medical service provider. The graphical user interface for modifying a captured image may digitally zoom or enlarge a captured image (or photograph).

The data interface may transmit the photograph to the patient's medical service provider in any suitable way. For example, the data interface may operate via data communications network. The data communications network may include a wireless protocol for exchanging data over a short distance personal area network (for example Bluetooth^{™} or Wi-Fi, which may be especially appropriate in a doctor's rooms (for example)) or may include coupling to the internet or to a telephone system (including via a mobile telephony network such as 3G or 4G data connection). In one example, the data interface may include a modem. The data interface may transmit the photograph by sending an email to the medical service provider. The data interface may transmit the photograph and also one or more of: the patient's name, the patient's address, the patient's telephone number, the patient's healthcare (or Medicare) number, an email address, the medical service provider's name, the medical service provider's email address, the medical service provider's address, symptoms experienced by the patient, and comments from the patient.

The medical service provider may have a database into which the captured photograph of the patient's orifice may be uploaded. In one embodiment, a user profile may be associated with a medical service provider profile. The user may choose to add a captured image to their profile for viewing by the medical service provider. The medical service provider's database may assist the provider to inspect the patient's orifice and/or to provide a record of how the patient's orifice appeared. The captured photograph of the patient's orifice may be automatically uploaded into the database.

As used herein, the term "medical service provider" may include any medical personnel who may need to inspect a photograph of a patient's orifice. Examples include doctors, nurses or audiologists. The medical service provider may be a telehealth or telemedicine service provider.

The graphical user interface may be configured to display a captured photograph and at least one photograph of an orifice having a known condition for comparison, to assist in identifying a condition affecting the patient's orifice. The graphical user interface may be so configured in any suitable way. For example, the graphical user interface may simultaneously display a captured photograph of the patient's orifice and at least one (especially one) photograph of an orifice having a known condition. The user may be able to replace (or cycle through) the photograph of an orifice having a known condition with a second photograph (or a plurality of photographs) of an orifice having a different known condition. The orifice inspection system may include a database including said at least one photograph of an orifice having a known condition. The at least one photograph of an orifice having a known condition may be a plurality of said photographs. The database may also include information on said conditions (as discussed further below).

In one embodiment, the orifice inspection system may include an image comparator for comparing the photograph of the patient's orifice with a database of photographs of orifices having known conditions. The image comparator may identify photographs displaying orifices having known conditions that are most likely to match (or correspond to) the photograph of the patient's orifice. The image comparator may display photographs of orifices having known conditions filtered by the likelihood of such a match. The image comparator may diagnose a condition affecting a patient's orifice.

The photographs of orifices having a known condition may include any condition affecting the orifice. The term "condition" includes diseases and disorders. For example, if the orifice is a patient's oral cavity, conditions may include one or more selected from the group consisting of: tonsillitis (especially acute tonsillitis), mononucleosis, pharyngitis, streptococcal pharyngitis (or strep throat), angina tonsillaris, candidiasis (or thrush) and measles. In another example, if the orifice is the ear, conditions may include one or more selected from the group consisting of: otitis externa, wax impaction, keratosis obturans, foreign bodies in the ear canal, osteoma and exostoses of the external ear canal. Otitis externa may include dermatological conditions (such as seborrhoeic dermatitis and atopic dermatitis), viral conditions (including Herpes zoster oticus), fungal conditions (otomycosis), acute localised otitis externa (furunculosis) and acute diffuse otitis externa.

The user may be able to obtain further information on any condition. For example, a user may be able to select (or click on) a photograph of an orifice having the condition to obtain further information. In one embodiment, such information includes one or more of: a summary of the condition, symptoms of the condition, the contagiousness of the condition, and recommendations of when to contact a medical service provider.

The orifice inspection system may include a data store (or database) of conditions affecting an orifice. The data store may include photographs of orifices having known conditions, as discussed above. The data store may also include information on known conditions, such as discussed in the preceding paragraph. The data store may be searchable in any suitable way. For example, the data store may be searchable by condition, or by symptoms. The data store may compile images captured by users. Images captured in the data store may be used for research and/or training purposes.

The orifice inspection system may include an orifice inspection device. The orifice inspection system may also include a light source for illuminating the orifice. The orifice inspection device may include a tongue depressor or an otoscope. The orifice inspection device may include a light source for illuminating the patient's orifice. The orifice inspection device may be an illuminated orifice inspection device. An exemplary illuminated tongue depressor is described in WO2012/021937. An exemplary otoscope is described in Australian Provisional Application No. 2016903740 (and the Patent Cooperation Treaty application derived from that application).

The orifice inspection device may be mountable relative to the image capture device, or the image capture device may be mounted on the orifice inspection device. In one embodiment, the orifice inspection system includes a user device including the image capture device, and wherein the orifice inspection device is mountable to the user device. In another embodiment, the orifice inspection device includes:
- a handle; and
- a coupler for coupling the handle to the user device;
wherein the coupler is releasably engageable with the handle; and wherein the coupler is fastenable to the user device. In one embodiment, the handle is slideable onto the coupler.

In another embodiment, the image capture device may be mounted on the orifice inspection device. The image capture device may be mounted on the orifice inspection device in any suitable way. In one embodiment, the image capture device may be integral with the orifice inspection device. For example, the handle of the orifice inspection device may have an inbuilt camera. In another embodiment, the image capture device may be a film, for example a film mounted to an orifice inserter of the orifice inspection device. In this embodiment, the image capture device and/or the orifice inserter may be for a single use.

In one embodiment, the orifice inspection system includes a handle for an orifice inspection device. The handle may be a handle for an illuminated tongue depressor or a handle for an otoscope. In another embodiment, the orifice inspection system includes an orifice inserter for insertion into a patient's orifice. The orifice inserter may be coupled to the handle. The orifice inserter may be adapted to direct light into the patient's orifice, or to a distal end of the orifice inserter. The orifice inserter may be the blade of a tongue depressor. The orifice inserter may be a speculum of an otoscope or a speculum or a device for illuminating a patient's nostril.

Accordingly, the orifice inspection device may include a handle and an orifice inserter. The orifice inserter may be configured to removably couple to the handle. The handle may include at least one light source. The orifice inspection device may be configured so that light emitted from the light source is transmitted by the orifice inserter into the patient's orifice. The light source in the handle may be activated when the handle and orifice inserter are coupled together.

When the orifice inspection device is used by a user, it may be held by the handle, and the orifice inserter may be inserted into the orifice. For example, if the orifice inserter is the blade of a tongue depressor, the term "blade" relates to the portion of the tongue depressor which may be used to depress the tongue of the patient. As used herein, the term "speculum", when used in relation to an otoscope or a device for inspecting a patient's nostril, refers to a portion of the orifice inspection device that is intended to contact a patient's ear or nostril. For example, at least part of the speculum is intended to contact the outer ear of the patient when the otoscope is in use. At least part of the speculum may be insertable into a patient's outer ear canal or nostril (for example). When the speculum is removable from the remainder of the otoscope or device for inspecting a patient's nostril, the term "speculum" refers to the entire removable portion, and not just to the portion of the device that is intended to contact the patient's orifice.

The shape of the orifice inserter will depend upon the orifice to be inspected. For example, if the orifice inspection device is a tongue depressor, the tongue depressor blade may be substantially laminar. The surface of the tongue depressor blade for depressing the tongue of the subject may be, for example, curved or flat.

The orifice inserter may be detachable. The orifice inserter may be sterilisable or disposable.

The orifice inserter may include a distal end portion (the portion at the end furthest from the handle), which may include a terminal end. The orifice inserter may include a proximate end portion (closest to the handle). The orifice inserter may be adapted to direct light from the light source to illuminate the orifice. In one embodiment, the orifice inserter is for (or is adapted to) focussing light from the light source to illuminate the orifice. In one embodiment, light from the light source may pass through the orifice inserter to illuminate the orifice.

The orifice inserter may direct light from the light source to illuminate the orifice in any suitable way. The orifice inserter may be configured (or adapted) to emit light from the terminal end. The orifice inserter may be made of a light-reflective material. The orifice inserter may include fibre optics extending from the terminal end to the proximate end. The fibre optics may be configured to transmit light from the light source to the distal end portion.

However, in a preferred embodiment the orifice inserter acts as an optical waveguide for light emitted from the light source. The orifice inserter may be relatively thin, which provides a shallow angle of incidence for light impinging on an inner surface of the upper and lower faces of the orifice inserter. This advantageously results in light travelling along the orifice inserter to undergo a minimal number of internal reflections within the orifice inserter so that light emitted from the light source, and especially substantially all light emitted from the light source, is emitted at the terminal end of the orifice inserter.

In some embodiments, the surface of the orifice inserter may be configured to control the passage of light. For example, the surface of the orifice inserter may be smooth. This may improve internal reflection for light passing through the orifice inserter, resulting in most, if not substantially all light passing through to the terminal end. Alternatively, the surface of the orifice inserter may be roughened where emission of light is desired. The thickness of the orifice inserter may also be used to control the passage of light, as the thinner the orifice inserter the more light is expected to pass through to the terminal end.

The orifice inserter may be configured to control where light is emitted. For example, it may be advantageous for substantially all light travelling through the orifice inserter to pass to the terminal end. Alternatively, it may be advantageous for some light to be emitted before the terminal end which may illuminate a greater portion of the orifice.

The orifice inserter may be a solid body extending from adjacent the light source to the terminal end. This may provide a waveguide for light emitted from the light source. The orifice inserter may include a reflective surface coating. Where light is to be emitted the orifice inserter may not include the reflective surface coating. Alternatively, the orifice inserter may include an internal cavity.

The orifice inserter may be transparent or clear. The orifice inserter may be made of any suitable materials which are capable of transmitting light. For example, the orifice inserter may be made of a plastic, especially poly(methyl methacrylate), polycarbonate or polystyrene (especially general purpose polystyrene); especially polycarbonate or a polystyrene. Advantageously, polycarbonate or polystyrene may be recyclable, optically transparent and not be brittle. The orifice inserter may especially be made from an injection moulded plastic.

The orifice inserter may be integral with the handle. The orifice inserter may be removable from the handle. The orifice inserter may be releasably engageable with the handle. When the orifice inserter is removable from the handle, the term "orifice inserter" refers to the entire removable portion, and not just to the portion of the orifice inserter that is insertable into the patient's orifice. At least a portion of the orifice inserter may be intended to contact the orifice of the patient when the orifice inspection device is in use.

The orifice inserter may be disposable. The orifice inserter may be sterilisable. In either case, the orifice inspection device may be handled hygienically. If the orifice inserter is sterilisable then a sterilisation method may be selected so as not to impair the function of the orifice inserter.

If the orifice inserter is removable from the handle, the proximate end portion of the orifice inserter may be engageable with an inserter-coupling portion of the handle. The proximate end portion may be coupled to the handle using a clip-fit, friction-fit, interference-fit or the like. The inserter-coupling portion of the handle may include a cavity into which the proximate end portion may be located, encapsulated or enveloped. Alternatively, the proximate end portion may include a cavity into which the inserter-coupling portion of the handle may be located, encapsulated or enveloped. The proximate end portion may include one or more projections or depressions which cooperate with corresponding depressions or projections on the handle inserter-coupling portion.

The handle may include a switch to connect a power supply to the light source. The switch may be multi-positionable, and may especially allow variable intensities of light to be emitted from the light source.

Coupling the orifice inserter to the handle may actuate the switch so that light emitted from the light source is transmitted by the orifice inserter to the orifice when the handle and orifice inserter are coupled together. Advantageously, this arrangement permits simple operation by a user, such as medical personnel or a member of the general public, by allowing the user to activate the light source by simply coupling the handle and orifice inserter together, thereby avoiding the need to actuate a separate switch and making the orifice inspection device easier to use.

In one embodiment, the orifice inserter proximate end portion includes a cavity and includes a projection within the cavity for actuating the switch when the handle and orifice inserter are coupled together. The switch may be positioned within a recess in the inserter-coupling portion of the handle, so that the orifice inserter cavity projection enters the recess and engages the switch when the handle and orifice inserter are coupled together. In this way the light source may be activated.

In another embodiment, the inserter-coupling portion of the handle includes a switch. The proximate end portion of the orifice inserter may include a cavity, and the inserter-coupling portion may be slideable inside the cavity. Sliding the inserter-coupling portion inside the cavity may actuate the switch. In this way the light source may be activated. The inserter-coupling portion may include an outer wall having a flap, and the flap may be positioned over the switch, so that depression of the flap (by coupling the inserter-coupling portion and the cavity) actuates the switch.

In a further embodiment, the handle inserter-coupling portion may include a cavity, and the proximate end portion of the orifice inserter may be slideable inside the cavity. Sliding the proximate end portion inside the cavity may actuate the switch. In this way the light source may be activated. The switch may be inside the cavity.

Alternatively, the switch may be located on the exterior of the handle, so that the switch may be actuated by the user after the handle and orifice inserter are coupled together.

The orifice inserter and handle may include at least one depression or projection to assist a user in handling the orifice inspection device, and in particular in decoupling the orifice inserter from the handle. For example, the orifice inserter and/or handle may include a plurality of projections or depressions, most especially a plurality of ridges. In one embodiment, the proximate end portion of the orifice inserter includes a plurality of ridges on at least one side wall opposite to the inner side wall of the cavity.

Advantageously, the presence of at least one depression or projection adjacent to the handle may allow a user to hygienically detach the orifice inserter from the handle without touching the portion of the orifice inserter that has come into contact with the patient's orifice. For example, the at least one depression or projection may be pushed or "flicked" by the user to detach the orifice inserter. This arrangement may also be relatively simple to manufacture.

The light source may be located within, or entirely within the handle. The handle may include a handle body, and the light source may be located within, or entirely within, the handle body. The light source may be located within, or entirely within, the inserter-coupling portion of the handle. The light source may be positioned adjacent to an opening in the inserter-coupling portion of the handle, so that visible radiation from the light source passes through the opening and into the orifice inserter, thereby maximising transmission of light into the orifice inserter.

The handle may include a plurality of light sources. In one embodiment, the handle includes 1, 2, 3, 4 or 5 light sources; especially three light sources. The light sources may be positioned so as to extend laterally across the handle. Including a plurality of spaced apart light sources may be advantageous, as this advantageously ensures that light enters the orifice inserter at a number of different locations, which can provide improved illumination and contrast when the orifice inspection device is used to illuminate the orifice of a patient.

Any suitable light source may be used in the orifice inspection device. Exemplary light sources include an incandescent bulb (including a halogen bulb), a fluorescent lamp, a high-intensity discharge lamp, a low-pressure sodium lamp, a light-emitting diode, a gas-discharge lamp and a monatomic gas bulb such as krypton or xenon. However, typically the light source or plurality of light sources in the handle is light-emitting diodes (LEDs), such as surface mount LEDs. LEDs typically use less energy than other forms of light source, thereby maximising battery life. Additionally, LEDs typically generate less heat than other forms of light source, thereby preventing the handle from overheating. Overheating may occur, for example, if incandescent or other light sources are used, and overheating may result in distortion of the handle and/or the orifice inserter, or patient discomfort when the orifice inspection device is used. The LEDs may be of about 3mm in size.

The light source may be connected to a power supply through the switch, especially on a circuit board. The circuit board may include other components, such as resistors and the like, although in one example, the circuit board consists of a light source, a switch and a connection for a power supply. The circuit may consist of, for example, three surface mounted LEDs in parallel, a switch in series and a battery.

The handle may include a power supply, such as a battery. Suitable batteries for use in the orifice inspection device may include a battery selected from: zinc-carbon, zinc-chloride, lithium, alkaline, nickel-cadmium, nickel-metal hydride, lead-acid and lithium ion, although any suitable power supply may be used. The battery may be especially a lithium-ion battery, more especially a CR2032 battery. The battery may be a rechargeable lithium battery. The handle may also include a removable cover for allowing the battery to be removed and replaced as required. The handle may also include a fastener for the cover, such as a screw to secure the cover closed. The use of such a fastener may be advantageous for child safety, for example.

In one example, the battery may be located within the portion of the handle opposite to the orifice inserter, however this is not essential and any suitable position may be used. The battery may be replaceable and/or rechargeable. For example, the battery may be charged by coupling the orifice inspection device to a power cord. In another example, the battery may be charged by wireless or inductive charging. In some examples, the battery in the orifice inspection device is not replaceable. For example, the handle may not include removable cover to access the battery, so that the handle is replaced when the battery is depleted. In this example, the outer shell of handle may be formed from a single piece of plastic, especially a single piece of injection moulded plastic.

In another example, the orifice inspection device may not include a battery. For example, the light source in the orifice inspection device may be powered by electricity from an external power supply when in use.

The handle may include a magnet. Advantageously this may allow the handle to be mounted on a metal surface such as a refrigerator or filing cabinet.

The handle may be made of a material such as a moulded plastic, especially a thermosetting plastic or a thermoplastic. The handle especially may be made from a material selected from: polystyrene, acrylonitrile butadiene styrene (ABS), polypropylene, polycarbonate and polymethylmethacrylate, especially acrylonitrile butadiene styrene (ABS). The handle may be especially made using an injection moulded plastic.

The handle may include a coupler for releaseably coupling the handle to the image capture device or user device. In one embodiment, the coupler is integral with the handle. The coupler may couple the handle to the image capture device or user device in any suitable way. For example, the coupler may couple to the image capture device or user device by virtue of suction, adhesive, hook and loop fasteners (such as Velcro^{™}) or mechanical fasteners (including couplers having arms biased to a closed position, clips, screws and the like). In one embodiment, the coupler includes a suction cap for affixing the handle to the user device or the image capture device. In another embodiment, the coupler includes a portion of a hook and loop fastener (the other portion of the hook and loop fastener may be adhered to a user device, for example). In another embodiment, the coupler may be configured to mechanically clamp the orifice inspection device, and/or to mechanically clamp the image capture device or user device including the image capture device. In a further embodiment, the coupler may include at least a pair of oppositely positioned arms biased to a closed position. The at least a pair of oppositely positioned arms may be biased to a closed position by a spring. The at least a pair of oppositely positioned arms may be adapted to engage with at least one side wall of a user device.

In another embodiment, the coupler is independent to the handle. The coupler may be releaseably coupleable to a handle of the orifice inspection device and/or releaseably coupleable to a user device or to an image capture device. The coupler may releaseably couple to the handle and/or to the user device or image capture device in any suitable way. For example, the coupler may couple to the image capture device or user device as outlined in the preceding paragraph. The coupler may couple to the handle by virtue of suction, adhesive, hook and loop fasteners (such as Velcro^{™}), mechanical fasteners (including couplers having arms biased to a closed position, clips, screws and the like) or a docking port or sleeve. In one embodiment, the coupler includes a handle engager for releaseably engaging the handle. The handle engager may include a cavity. An end of the handle may be insertable into the handle engager. The handle engager may be releaseably engageable to the handle by clip-fit, friction-fit, interference-fit or the like. The handle engager may be planar. The handle engager may be slideably engageable with the handle. The handle may include a groove for engaging the handle. The handle engager may be fastenable to an image capture device or to a user device. For example, the handle engager may include an adhesive layer for fastening the handle engager. An exemplary adhesive is VHB Tape PX5011, manufactured by 3M. The adhesive may be compatible with hydrophobic, hydrophilic or amphiphilic surfaces. The adhesive layer may be of any suitable thickness. In one embodiment, the adhesive layer is of less than 1.5 mm thickness, especially about 1.1 or 0.8 mm thickness.

The handle, orifice inserter and/or coupler may be manufactured and/or sold separately. In either case, the orifice inserter may be sold in a sterile form, for example provided sterilised in packaging, allowing the orifice inserter to be used as a single use disposable item. The orifice inserter may alternatively be sterilisable for repeated use. The handle and/or coupler may also be sterilisable, although in a preferred form the handle and coupler do not require sterilisation and may be repeatedly used. If the handle is sterilisable, and the handle includes a power supply and a light source, then the power supply and the light source should be selected and located so that these components will continue to function after repeated sterilisation (whether chemical or thermal sterilisation, for example). For example, it may be advantageous to employ inductive charging if the orifice inspection device is sealed for sterilisation.

In a further example, the orifice inserter is integrally formed with handle. In this example, the orifice inserter is not removably coupled to the handle, and the outer shell of orifice inserter and handle may be formed from a single piece of plastic, especially a single piece of injection moulded plastic. In this example, the orifice inspection device may be sterilisable for repeated use, or may be sold in a sterile form, for example provided sterilised in packaging, allowing the orifice inspection device to be used as a single use disposable item.

In some examples, the handle includes a keyring attachment, allowing the handle to be easily carried by a user. The handle may then be easily attached to a orifice inserter when the orifice inspection device is to be used.

The orifice inserter and handle may be of any suitable length or shape. In one embodiment, the length of handle alone may be, for example, 15 to 65 mm, especially from 20 mm to 60 mm, or from 25 to 55 mm, more especially from 30 to 50 mm, or from 35 mm to 45 mm, most especially about 40 mm. When present, the length of the orifice inserter-coupling portion of the handle may be, for example, from 5 to 35 mm, especially from 10 to 30 mm, more especially from 15 mm to 25 mm, most especially about 20 mm.

In another example, the width of the orifice inspection device (including the orifice inserter and/or the handle) is from 5 mm to 40 mm, more especially from 7 mm to 35 mm, more especially from 10 mm to 30 mm, more especially from 15 mm to 25 mm, most especially about 20 mm.

In another example, the height of the handle 4 is from 0.5 mm to 70 mm, especially from 3 mm to 35 mm, more especially from 5 mm to 20 mm, more especially from 6 mm to 12 mm, more especially about 9 mm.

In one embodiment, the orifice inspection device includes:
a. A handle which includes:
   (i). A light source; and
   (ii).A switch for connecting a power supply to the light source; and
b. A orifice inserter for illuminating the orifice, the orifice inserter being removably coupled to the handle and having a cavity at one end for receiving the handle;
wherein coupling the orifice inserter to the handle actuates the switch so that light emitted from the light source is transmitted by the orifice inserter to the orifice.

In another embodiment, the orifice inspection device includes:
a. A handle having a handle body, wherein the handle includes:
   i. A light source entirely within the handle body; and
   ii. An inserter-coupling portion including a switch for connecting a power supply to the light source; and
b. A orifice inserter for illuminating the orifice, the orifice inserter being removably coupled to the handle and including:
   i. A cavity at one end for receiving the inserter-coupling portion of the handle, and
   ii. A solid body extending from the cavity to the end distal the cavity, wherein the solid body is configured to act as an optical waveguide for light emitted from the light source;
wherein coupling the orifice inserter to the handle actuates the switch so that light emitted from the light source is transmitted by the orifice inserter into the orifice.

The orifice inspection device may include a lens for magnifying the view of the patient's orifice. The handle may include the lens, or the orifice inserter may include the lens. The orifice inserter may include a lens or a lens engager for engaging the lens. The orifice inserter may be integral with the lens or lens inserter.

In one embodiment, the orifice inspection device may be adapted to inspect more than one type of orifice (for example, the outer ear and the oral cavity). In this embodiment, a plurality of orifice inserters for inspecting different orifices may be coupleable to a handle. In this way, a user may use the same orifice inspection system to inspect two, three or more of a patient's orifices (for example the outer ear, nostril and the oral cavity) by simply attaching a different orifice inserter to the orifice inspection device handle.

In a fifth aspect the present invention relates to an orifice inspection device. The orifice inspection device may be as described above. The orifice inspection device may include a handle and an orifice inserter. The handle may include a light source. The orifice inspection device may be an illuminated orifice inspection device. The orifice inspection device may be for illuminating the orifice of a patient. The orifice inspection device may be configurable to inspect the patient's outer ear and oral cavity.

In an embodiment of the fifth aspect, there is provided an orifice inspection device including:
- A handle including a light source;
- An orifice inserter for illuminating a patient's orifice, wherein the orifice inserter extends from the handle; and
- A coupler fastenable to a user device, wherein the user device includes an image capture device, and wherein the coupler is releasably engageable with the handle.

In a sixth aspect, the present invention relates to a handle of an orifice inspection device. The handle of the orifice inspection device may be as described above.

In a seventh aspect, the present invention relates to an orifice inserter for an orifice inspection device. The orifice inserter may be as described above.

In embodiments of the first to fourth aspects of the present invention, the orifice inspection system further includes a positioner for positioning the image capture device relative to the orifice inspection device. In one embodiment, the positioner is a coupler for coupling the image capture device relative to the orifice inspection device (especially for coupling the image capture device to the orifice inspection device). In an embodiment of the fifth aspect of the present invention, the orifice inspection device further includes a coupler for coupling the image capture device relative to the orifice inspection device (especially for coupling the image capture device to the orifice inspection device). In one embodiment, the coupler is releaseably engageable with the handle of the orifice inspection device. In another embodiment, the coupler is integral with the handle of the orifice inspection device. Accordingly, in an embodiment of the sixth aspect, the handle includes a coupler for coupling the image capture device to the handle. In one embodiment, the coupler is releasably engageable with an image capture device.

In an eighth aspect, the present invention relates to a coupler for coupling the image capture device or user device relative to the orifice inspection device. The coupler may be for coupling the image capture device or user device to the orifice inspection device. The coupler may be releaseably engageable with a handle of an orifice inspection device. The coupler may be releaseably engageable with an image capture device.

Features of the fifth, sixth, seventh and eighth aspects of the present invention may be as described for the first to fourth aspects of the present invention.

In a ninth aspect, not falling within the scope of the claimed invention, the present disclosure relates to a patient orifice inspection method, including:
- Positioning, using an orifice positioning guide, an image capture device in relation to an orifice of a patient; and
- Capturing, using the image capture device, a photograph of the orifice of the patient.

In one embodiment, a graphical user interface may include the orifice positioning guide. In one embodiment, the method may include the step of transmitting, using a data interface, the photograph to the patient's medical service provider. The method may further include the step of comparing the captured photograph and at least one photograph of an orifice having a known condition, to thereby assist in identifying a condition affecting the patient's orifice. The method may further include the step of retrieving information of at least one known condition affecting an orifice.

Features of the ninth aspect not falling within the scope of the claimed invention, may be as described for the first to fourth aspects of the present disclosure.

In a tenth aspect, not falling within the scope of the claimed invention, the present disclosure relates to a patient orifice inspection method, including:
- Capturing, using an image capture device, a photograph of an orifice of a patient; and
- Transmitting, using a data interface, the photograph to the patient's medical service provider.

In one embodiment, the method may include the step of positioning, using an orifice positioning guide, an image capture device in relation to an orifice of a patient. A graphical user interface may include the orifice positioning guide. The method may further include the step of comparing the captured photograph and at least one photograph of an orifice having a known condition, to thereby assist in identifying a condition affecting the patient's orifice. The method may further include the step of retrieving information of at least one known condition affecting an orifice.

Features of the tenth aspect, not falling within the scope of the claimed invention, may be as described for the first to fourth aspects of the present disclosure.

In an eleventh aspect, not falling within the scope of the claimed invention, the present disclosure relates to a patient orifice inspection method, including:
- Capturing, using an image capture device, a photograph of an orifice of a patient; and
- Comparing the captured photograph and at least one photograph of an orifice having a known condition, to thereby assist in identifying a condition affecting the patient's orifice.

In one embodiment, the method may include the step of positioning, using an orifice positioning guide, an image capture device in relation to an orifice of a patient. A graphical user interface may include the orifice positioning guide. The method may further include the step of transmitting, using a data interface, the photograph to the patient's medical service provider. The method may further include the step of retrieving information of at least one known condition affecting an orifice.

Features of the eleventh aspect not falling within the scope of the claimed invention, may be as described for the first to fourth aspects of the present disclosure.

In a twelfth aspect, not falling within the scope of the claimed invention, the present disclosure relates to a method of illuminating the orifice of a patient, the method including the step of inserting at least a portion of an orifice inspection device into a patient's orifice, or contacting at least a portion of an orifice inspection device with a patient's orifice. Features of the orifice inspection device of the twelfth aspect of the present disclosure may be as described for the fifth aspect of the present disclosure.

In a thirteenth aspect, not falling within the scope of the claimed invention, the present disclosure relates to a method of illuminating the orifice of a patient, the method including the step of coupling the orifice inserter to the handle to form the orifice inspection device of the fifth aspect of the present invention. This step may thereby actuate the switch and activate the light source. In one embodiment, the method further includes the step of inserting at least a portion of an orifice inspection device into a patient's orifice, or contacting at least a portion of an orifice inspection device with a patient's orifice.

In a fourteenth aspect, not falling within the scope of the claimed invention, the present disclosure relates to a method of coupling an image capture device or user device to a orifice inspection device, the method including the step of coupling an image capture device or user device to the coupler. The method may also include the step of coupling an orifice inspection device to the coupler. Features of the fourteenth aspect of the present disclosure may be as described for the first to thirteenth aspects of the present disclosure.

In a fifteenth aspect, not falling within the scope of the claimed invention, the present disclosure relates to an orifice inspection system including an image capture device for capturing a photograph of a patient's orifice; wherein the orifice inspection system is configured to detect the presence of an orifice inspection device, and to automatically adjust the image magnification of the image capture device based on the location of the orifice inspection device. Features of the fifteenth aspect of the present disclosure may be as described for the first to fourth aspects of the present disclosure.

In a sixteenth aspect, not falling within the scope of the claimed invention, the present disclosure relates to a patient orifice inspection method including:
- Detecting the presence of an orifice inspection device;
- Automatically adjusting the image magnification of the image capture device based on the location of the orifice inspection device; and
- Capturing a photograph of the patient's orifice

Features of the sixteenth aspect, not falling within the scope of the claimed invention, may be as described for the first to fourteenth aspects of the present disclosure.

In a seventeenth aspect, not falling within the scope of the claimed invention, the present disclosure relates to a patient orifice inspection method including:
- Mounting an orifice inspection device relative to an image capture device;
- Illuminating a patient's orifice with the orifice inspection device; and
- Capturing a photograph of the patient's orifice as illuminated by the orifice inspection device.

In one embodiment of the seventeenth aspect, the step of mounting the orifice inspection device relative to an image capture device is a step of mounting the orifice inspection device to a user device, and the user device includes the image capture device. In a further embodiment, the orifice inspection device includes:
- a handle; and
- a coupler for coupling the handle to the user device;
wherein the step of mounting the orifice inspection device to the user device is a step of coupling the handle to the coupler, wherein the coupler is fastened to the user device.
In another embodiment, the step of coupling the handle to the coupler is a step of sliding the handle onto the coupler.

In one embodiment of the seventeenth aspect, before the photograph is captured the method further includes the step of detecting the presence of the orifice inspection device, and automatically adjusting the image magnification of the image capture device based on the location of the orifice inspection device. In one embodiment, the step of detecting the presence of the orifice inspection device is a step of a user confirming that the orifice inspection device is present.

In a further embodiment of the seventeenth aspect, the method further includes the step of using an orifice positioning guide to position the image capture device in relation to the patient's orifice. In another embodiment, the method further includes the step of transmitting the captured photograph to the patient's medical service provider. In a further embodiment, the method further includes the step of comparing the captured photograph and at least one photograph of an orifice having a known condition, to thereby assist in identifying a condition affecting the patient's orifice. In another embodiment, the orifice is the patient's oral cavity, outer ear or nostril.

Features of the seventeenth aspect of the present disclosure may be as described for the first to sixteenth aspects of the present disclosure.

Any of the features described herein can be combined in any combination with any one or more of the other features described herein within the scope of the invention.

### BRIEF DESCRIPTION OF DRAWINGS

Examples of the invention will now be described by way of example with reference to the accompanying Figures, in which:
Figure 1 illustrates an orifice inspection system, according to one embodiment of the present invention;
Figure 2 shows a screenshot of a main menu screen of an application of the system of Figure 1, according to an embodiment of the present invention;
Figure 3 is a screenshot of the system of Figure 2, illustrating a graphical user interface showing an image to be captured by an image capture device, with an orifice positioning guide overlaid on the image;
Figure 4 is a screenshot of the system of Figure 2, illustrating a photograph captured by the image capture device;
Figure 5 is a screenshot of the system of Figure 2, illustrating a graphical user interface displaying the captured photograph and a photograph of an orifice having a known condition for comparison;
Figure 6 is a screenshot of the system of Figure 2, illustrating further information concerning an orifice having a known condition;
Figure 7 is a screenshot of the system of Figure 2, illustrating an image to be transmitted to a medical service provider;
Figure 8 is a screenshot of the system of Figure 2, illustrating a database of known conditions affecting an orifice, browseable by photograph;
Figure 9 is a screenshot of the system of Figure 2, illustrating a database of known conditions affecting an orifice, browseable by symptom;
Figure 10 illustrates a patient orifice inspection method according to an embodiment of the present invention;
Figure 11 shows a perspective view of a handle of an example orifice inspection device according to an embodiment of the present invention;
Figure 12 shows a side view of the handle of Figure 11;
Figure 13 shows a top view of the handle of Figure 11;
Figure 14 shows a perspective view of a tongue depressor according to an embodiment of the present invention;
Figure 15 shows a perspective view of the tongue depressor of Figure 14, with the blade decoupled from the handle;
Figure 16 shows a side view of the tongue depressor of Figure 14;
Figure 17 shows a side view of the tongue depressor of Figure 15;
Figure 18 shows a top view of the tongue depressor of Figure 14;
Figure 19 shows a top view of the tongue depressor of Figure 15;
Figure 20 shows a perspective view of the tongue depressor of Figure 14, a coupler and a user device coupled together;
Figure 21 shows a side view of the tongue depressor, coupler and user device of Figure 20;
Figure 22 shows an exploded perspective view of the tongue depressor, coupler and user device of Figure 20;
Figure 23 shows an exploded side view of the tongue depressor, coupler and user device of Figure 20;
Figure 24 shows a perspective view of a tongue depressor coupled to a user device according to an embodiment of the present invention;
Figure 25 shows a side view of the tongue depressor and user device of claim 24;
Figure 26 shows an exploded perspective view of the tongue depressor and user device of claim 24;
Figure 27 shows an exploded side view of the tongue depressor and user device of claim 24;
Figure 28 shows an exploded perspective view of an otoscope coupled to a user device according to an embodiment of the present invention;
Figure 29 shows a top view of an otoscope according to an embodiment of the present invention;
Figure 30 shows a side view of the otoscope of Figure 29;
Figure 31 shows a bottom view of the otoscope of Figure 29;
Figure 32 shows an exploded side view of the otoscope of Figure 29;
Figure 33 shows an exploded perspective view of the otoscope of Figure 29;
Figure 34 shows an exploded perspective view of a tongue depressor according to an embodiment of the present invention;
Figure 35 shows a front view of the tongue depressor of Figure 34;
Figure 36 shows a cross-sectional view through section P-P of Figure 39;
Figure 37 shows a cross sectional view through section O-O of Figure 35;
Figure 38 shows a cross sectional view through section M-M of Figure 35;
Figure 39 shows a cross sectional view through section L-L of Figure 35;
Figure 40 shows an detailed view of section N of Figure 39;
Figure 41 shows an exploded perspective view of a tongue depressor according to an embodiment of the present invention;
Figure 42 shows a front view of the tongue depressor of Figure 34;
Figure 43 shows a cross-sectional view through section E-E of Figure 42;
Figure 44 shows a cross sectional view through section C-C of Figure 42;
Figure 45 shows a cross sectional view through section F-F of Figure 44;
Figure 46 shows a cross sectional view through section D-D of Figure 44;
Figure 47 shows a left side view of a coupler fastened to a user device according to an embodiment of the present invention;
Figure 48 shows a front view of the coupler fastened to a user device of Figure 47;
Figure 49 shows a right side view of the coupler fastened to a user device of Figure 47;
Figure 50 shows a perspective view of the coupler fastened to a user device of Figure 47;
Figure 51 shows a left side view of a handle mounted to a user device according to an embodiment of the present invention;
Figure 52 shows a front view of the handle mounted to a user device of Figure 51;
Figure 53 shows a right side view of the handle mounted to a user device of Figure 51;
Figure 54 shows a perspective view of the handle mounted to a user device of Figure 51;
Figure 55 illustrates an orifice inspection system, according to an embodiment of the present invention;
Figure 56 shows a screenshot of an application of the system of Figure 55, illustrating a photograph captured by the image capture device;
Figure 57 shows a screenshot of the system of Figure 56, illustrating the photograph taken in Figure 56 as modified by a user;
Figure 58 shows a screenshot of the system of Figure 56, illustrating a screenshot showing the photograph being assigned to a user profile;
Figure 59 shows a screenshot of the system of Figure 56, illustrating a menu of actions that can be taken after the image capture is complete;
Figure 60 shows a screenshot of the system of Figure 56, illustrating a graphical user interface displaying the captured photograph and a photograph of an orifice having a known condition for comparison; and
Figure 61 illustrates a patient orifice inspection method according to an embodiment of the present invention.

Preferred features, embodiments and variations of the invention may be discerned from the following Description which provides sufficient information for those skilled in the art to perform the invention. The following Description is not to be regarded as limiting the scope of the preceding Summary of the Invention in any way.

### DESCRIPTION OF EMBODIMENTS

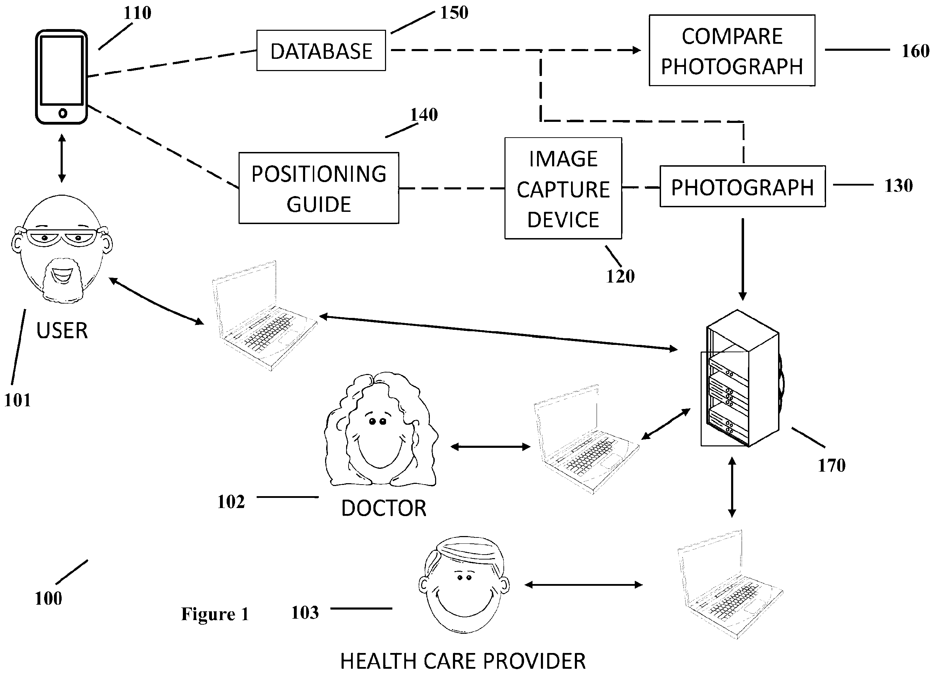

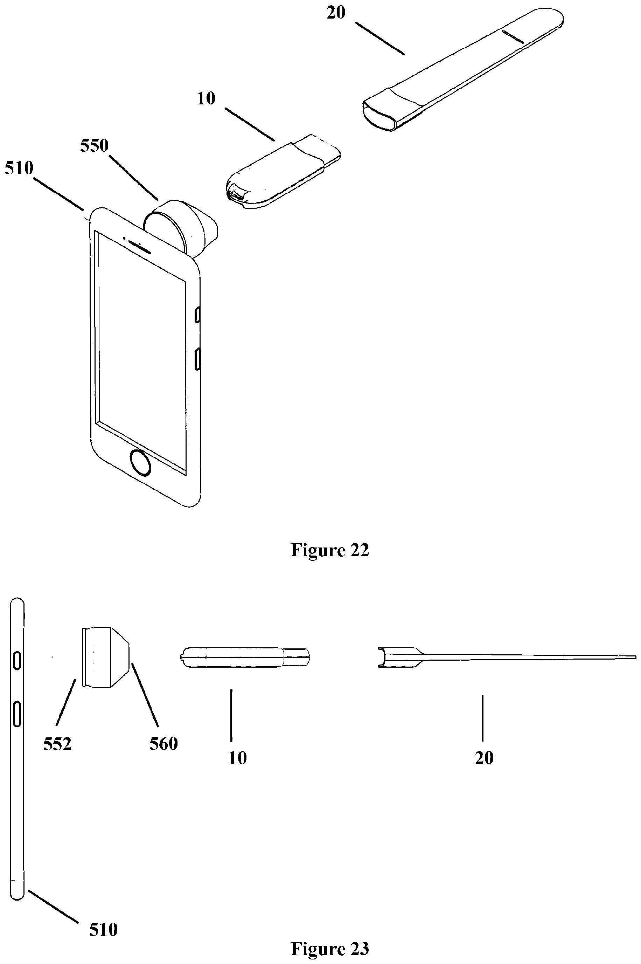

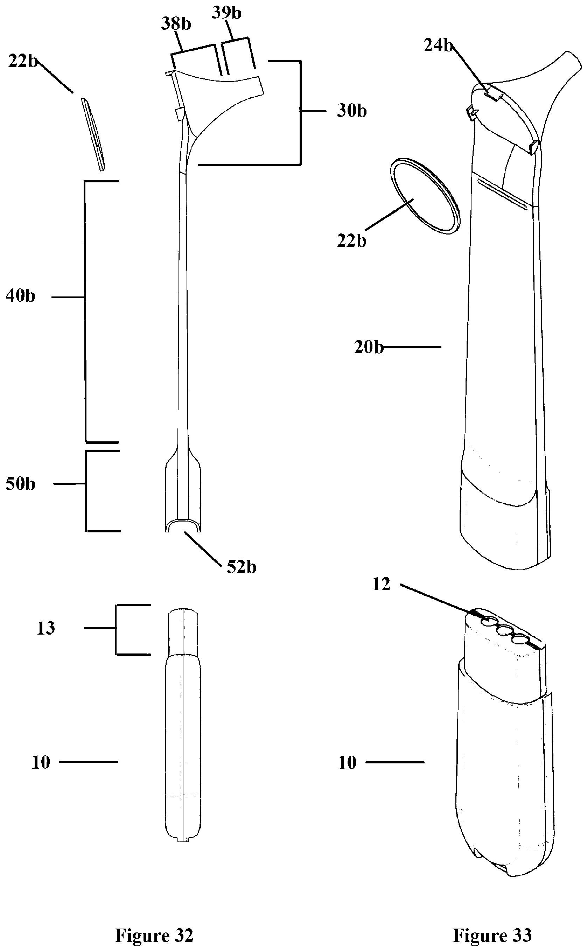

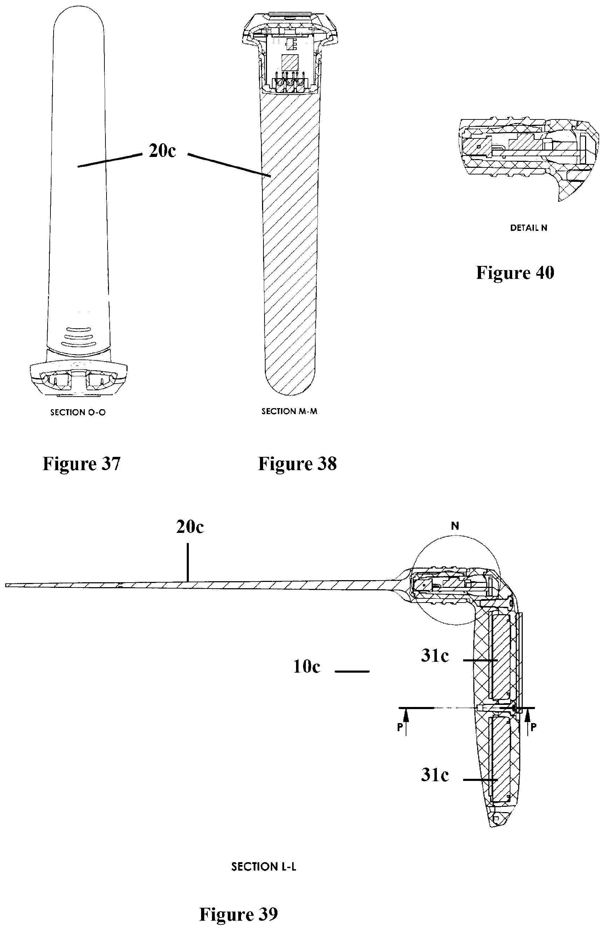

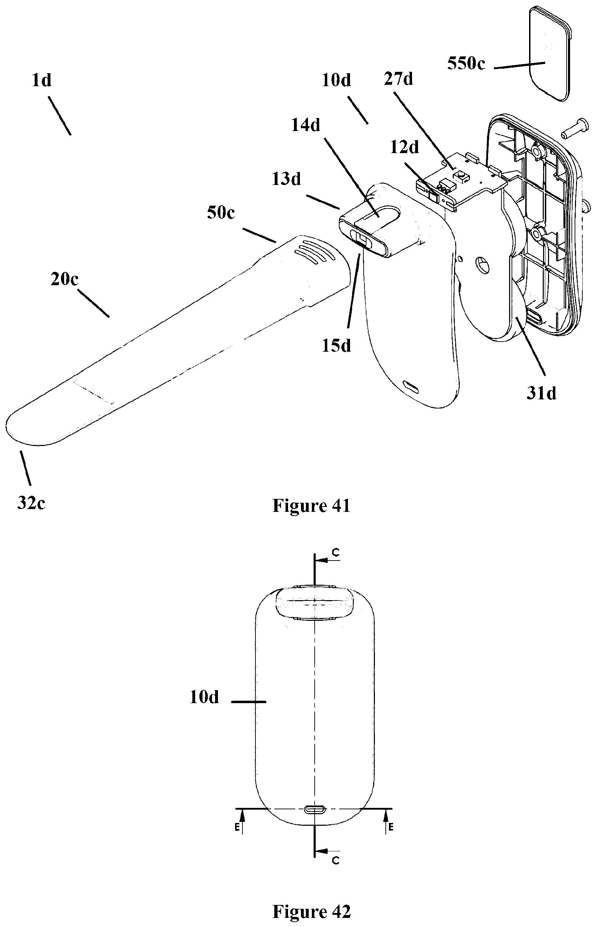

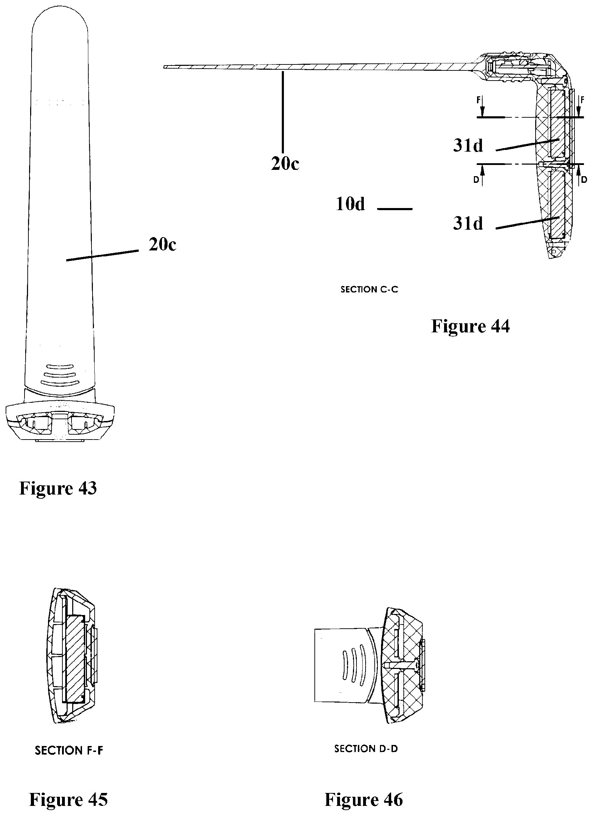

Embodiments of the present invention will now be described with reference to Figures 1 to 61. In the figures, like reference numerals refer to like features.

Figure 1 illustrates an example orifice inspection system 100, according to an embodiment of the present invention. The orifice inspection system 100 may be useable domestically (for example, by a patient or a family member of the patient), or by a medical professional (for example in a doctor's surgery or hospital).

As an example, a user 101 may have an application on their computer or smartphone 110. The application uses an image capture device 120 (such as a camera or webcam) to capture a photograph 130 of a patient's orifice. The application includes a graphical user interface which includes a positioning guide 140 for positioning a patient's orifice in the photograph 130 captured by the image capture device 120. A database 150 of at least one photograph of an orifice having a known condition may be accessible via the graphical user interface. The graphical user interface may be used to compare the photograph 130 with images from the database at 160. For example, the graphical user interface may be configured to display the captured photograph 130 and at least one photograph of an orifice having a known condition for comparison, to assist in identifying a condition affecting the patient's orifice.

The user 101 may use a data interface on the computer or smartphone 110 in order to transmit the photograph to the patient's medical service provider. For example, the data interface may transmit the photograph 130 by sending an email to the medical service provider. The data interface may also transmit the photograph 130 by uploading the photograph onto a server 170, from which one or more of the user 101 or the medical service provider (such as a doctor 102, or other health care provider 103) may view the photograph 130.

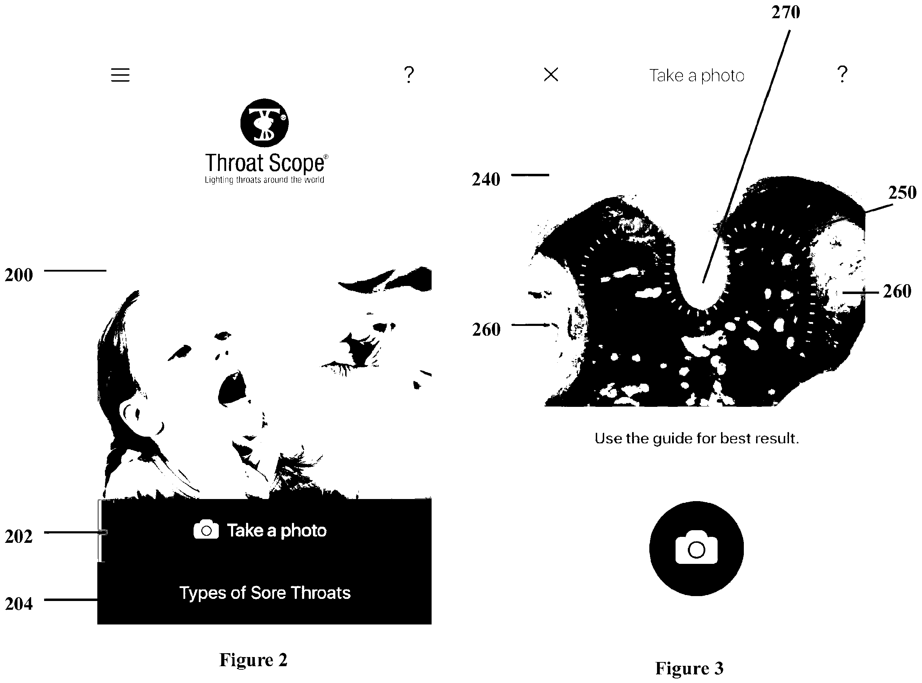

An exemplary orifice inspection system for the oral cavity will now be described with reference to Figures 2 to 9. The orifice inspection system exemplified in Figures 2 to 9 includes an application for a smartphone.

Figure 2 illustrates a main menu screen 200 for the application. From this screen the user can choose to take a photograph of an oral cavity 202, or browse a database showing at least one oral cavity having a known condition 204.

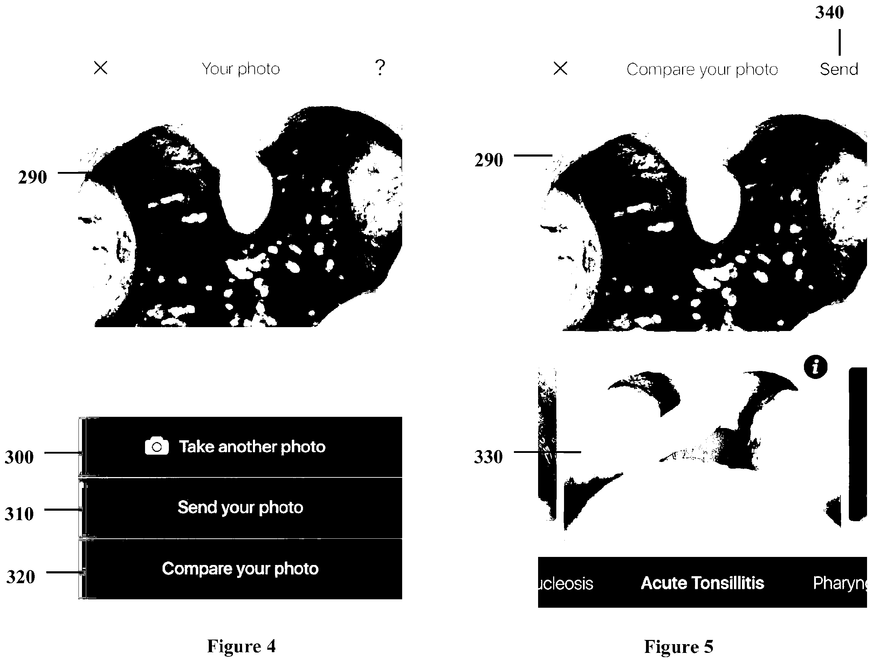

If the user choses to take a photograph 202, a graphical user interface is provided showing an image 240 to be captured by an image capture device (see Figure 3). Overlaid on image 240 is an orifice positioning guide 250. The orifice positioning guide 250 is a broken line illustrating the correct position of the palatine tonsils 260 and the uvula 270. When the image 240 is in the correct position, the user can capture the photograph 290, as shown in Figure 4.

The user then has the option of capturing another photograph 300, transmitting the captured photograph to a medical service provider 310, or comparing the captured photograph with photographs of oral cavities having known conditions 320.

If the user chooses to compare the photograph, the graphical user interface displays the captured photograph 290, and at least one photograph of an oral cavity having a known condition 330 (see Figure 5). In Figure 5, the user is able to change the photograph of the oral cavity having a known condition 330 to compare photographs of acute tonsillitis, mononucleosis, pharyngitis, and streptococcal pharyngitis. The user may click on the image of the oral cavity having a known condition 330 to obtain further information on the condition.

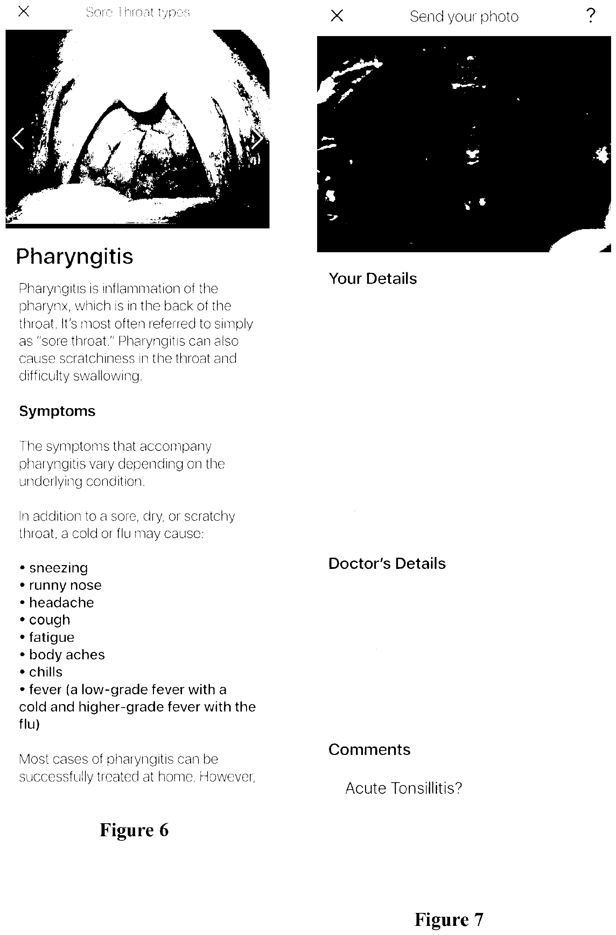

Figure 6 illustrates an example of further information which may be obtained by the user by clicking on the image of the oral cavity having a known condition. The further information includes: a summary of the condition, symptoms of the condition, the contagiousness of the condition, and recommendations of when to contact a medical service provider.

If the user chooses to send the captured photograph 290 to a medical service provider, they can do so either prior to or after comparing the captured photograph 290 with photographs of oral cavities having known conditions (see Figure 4 at 310 and Figure 5 at 340). If this option is chosen, the user may be directed to a form into which information may be entered concerning the patient's name, the patient's address, the patient's phone number, the patient's healthcare (or Medicare) number, an email address, the medical service provider's name, the medical service provider's email address, the medical service provider's address, symptoms experienced by the patient, and comments from the patient. An exemplary form is illustrated in Figure 7.

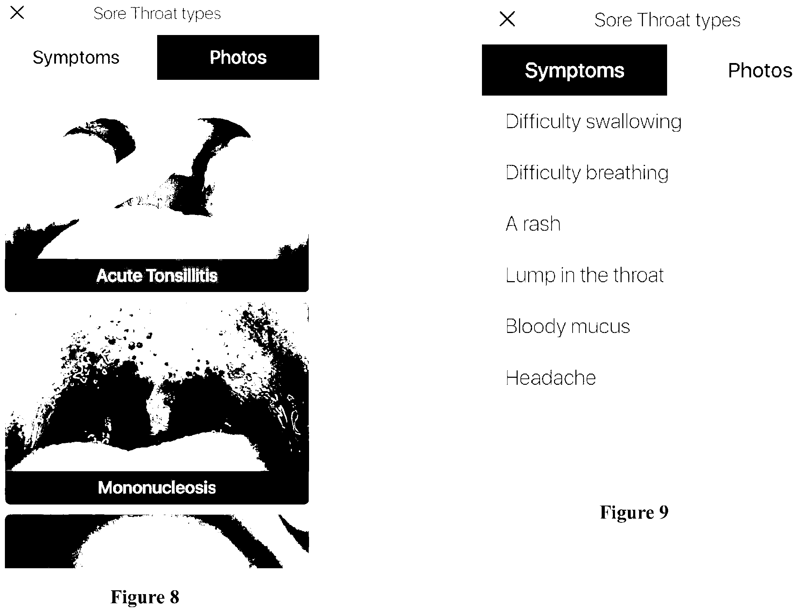

Alternatively, the user can browse oral cavities having known conditions via 204 at Figure 2. Choosing this option allows the user to access a database of oral cavities having known conditions, and this database is browseable by condition (Figure 8) or by symptom (Figure 9). Again, selecting a particular condition allows the user to access further information on that condition, as illustrated in Figure 6.

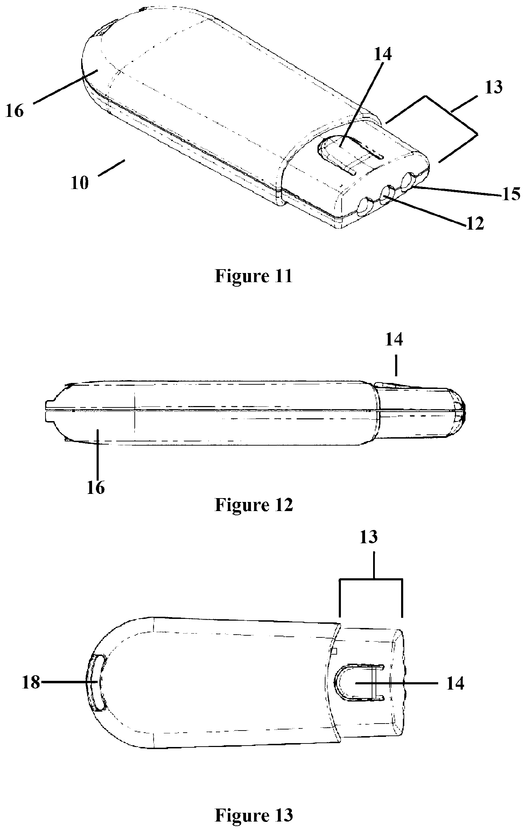

The orifice inspection system may include the application of Figures 2-9 and an orifice inspection device 1 (see Figures 11 to 19 and 28-54). The orifice inspection device 1 may be for illuminating the patient's orifice which in turn allows for a clearer photo to be captured by the image capture device.

The orifice inspection device 1 of the embodiment of Figures 11-19 is a tongue depressor. Features of the exemplary tongue depressor of Figures 11-19 are as outlined below.

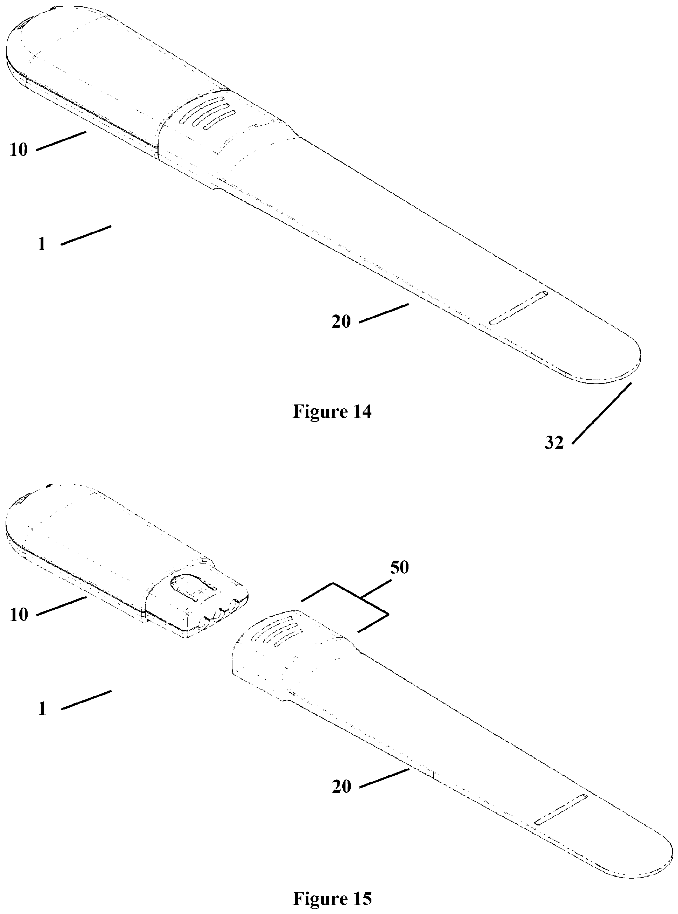

The orifice inspection device 1 includes a handle 10 (see Figures 11-19) and a blade 20 (see Figures 14-19). The tongue depressor 1 is for illuminating the oral cavity, especially the pharynx, at least one tonsil and the uvula.

The blade 20 extends from the handle 10 and is for directing light from the light source 12 (see Figure 11 and as discussed below) to illuminate the oral cavity.

The handle 10 of the tongue depressor is illustrated in Figures 11-13. As shown in Figure 11, the handle 10 includes three light sources 12. Light from the light source 12 passes through the blade 20 to illuminate the oral cavity.

The blade 20 in Figures 14-19 is releasably engageable from the handle 10. The blade 20 acts as an optical waveguide for light emitted from the light source 12. The blade 20 is a solid body extending from adjacent the light source 12 to its terminal end 32. The blade 20 of Figures 14-19 is adapted to emit light from the terminal end 32. The blade 20 may be made from injection moulded plastic. The blade 20 may be disposable.

The blade 20 includes a distal end portion 30 having a terminal end 32. The distal end portion 30 is substantially flat in a longitudinal and lateral direction.

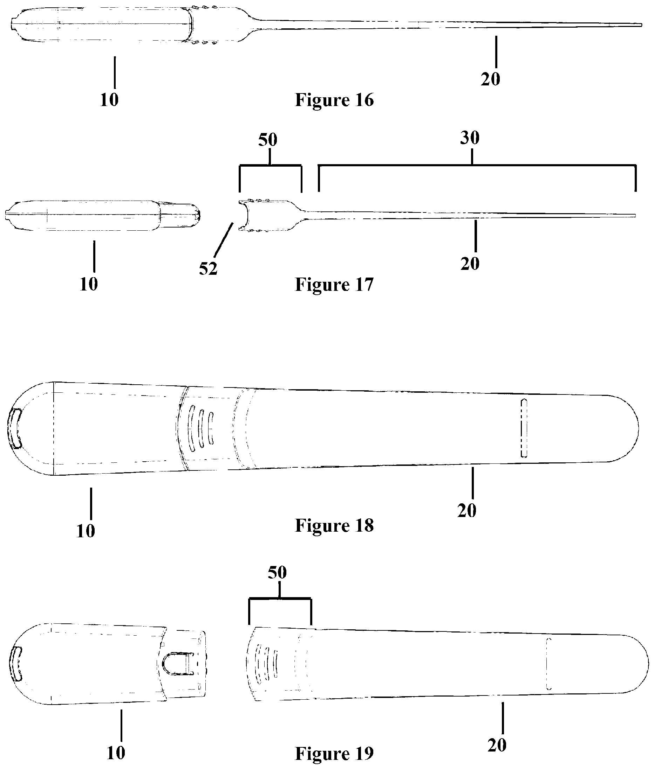

At least a portion of the distal end portion 30 is intended to be inserted into the patient's oral cavity. The blade 20 also includes a proximate end portion 50 (see Figures 15, 17 and 19). The proximate end portion 50 of the blade 20 is slideably engageable with the insert-coupling portion 13 of the handle 10 (see Figures 11 and 13). The proximate end portion 50 includes a cavity 52 into which the insert-coupling portion 13 of the handle is located (see Figure 17). The internal walls of the cavity 52 are smooth.

The handle 10 includes a switch to connect a power supply (not shown) to the light source 12. Coupling the blade 20 to the handle 10 actuates the switch so that light emitted from the light source 12 is transmitted by the blade 20 to the oral cavity when the handle 10 and blade 20 are coupled together.

The insert-coupling portion 13 includes an outer wall having a flap 14, and the flap 14 is positioned over the switch, so that depression of the flap 14 by sliding the insert-coupling portion 13 into cavity 52 actuates the switch and activates the light source 12.

The light source 12 is located entirely within the handle 10. The light source is positioned adjacent to an opening 15 in the insert-coupling portion 13 so that light from the light source 12 passes through the opening 15 and into the blade 20. The handle 10 includes three light sources 12 which are positioned to extend laterally across the handle 10. The light sources 12 are especially light-emitting diodes (LEDs).

The power source in the handle 10 is in the form of a battery. The battery is especially a lithium-ion battery. The battery is connected to the light source 12 and the switch via a circuit board (not shown). The handle 10 includes a removable cover 16 for allowing the battery to be removed and replaced, as required. The removable cover 16 includes an aperture 18 providing an attachment for a keyring. The handle 10 may be made from injection moulded plastic.

The handle 10 especially does not require sterilisation and is intended for multiple uses. The blade 20 is either sterilisable (e.g. via heat or chemical sterilisation), or is disposable. The blade 20 and the handle 10 may be sold separately.

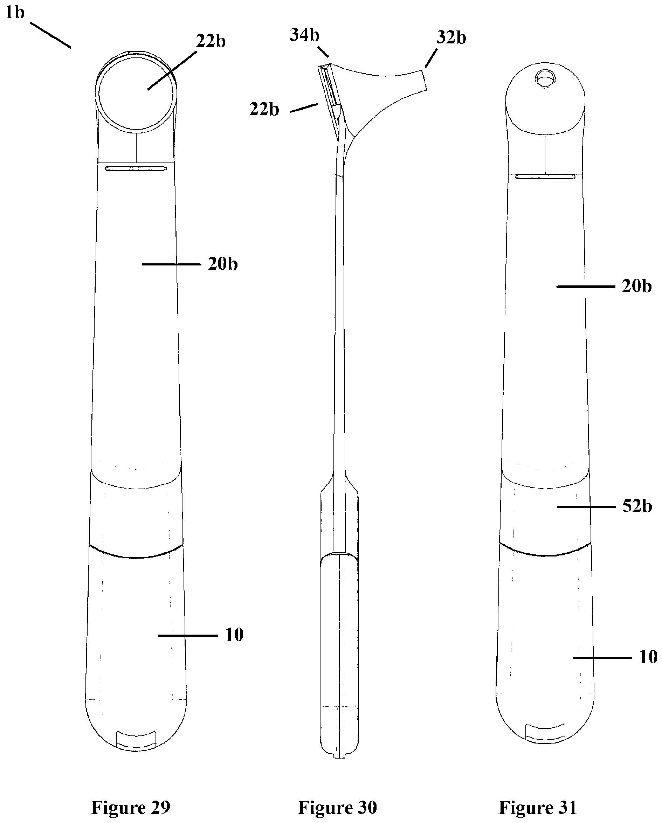

The orifice inspection device 1b of the embodiment of Figures 29-33 is an otoscope. Features of the exemplary otoscope 1b of Figures 29-33 are as outlined below.

The handle 10 for otoscope 1b is as described above. Speculum 20b is slideably engageable with the attachment-coupling portion 13 of the handle 10.

Speculum 20b acts as an optical waveguide for light emitted from the light source 12, and is a solid body extending from adjacent the light source 12 to its terminal end 32b. Speculum 20b is made from clear injection moulded plastic and is disposable.

Speculum 20b includes a distal end portion 30b having a terminal end 32b and a non-terminal end 34b (see Figure 30). At least a portion of the distal end portion 30b is intended to be inserted into the patient's outer ear when the otoscope 1b is used. The distal end portion 30b includes a side wall having a substantially conical portion 38b extending from the non-terminal end 34b, and a substantially cylindrical portion 39b extending from the terminal end 32b. The side wall 38b, 39b defines a visualisation passageway through the distal end portion 30b. The distal end portion 30b defines an aperture at the terminal end 32b and includes a lens engager 24b at the non-terminal end 34b. The lens 22b is releaseably engageable with the lens engager 24b. Once fitted, the lens 22b is positioned proximate to the non-terminal end 34b.

Speculum 20b also includes a medial portion 40b and a proximate end portion 50b. The medial portion 40b is substantially flat in a lateral and longitudinal direction. The longitudinal axis of the distal end portion 30b is at about 75° relative to the longitudinal axis of the handle 10.

The proximate end portion 50b of the speculum is slideably engageable with the attachment-coupling portion 13 of the handle 10. The proximate end portion 50b includes a cavity 52b into which the attachment-coupling portion 13 of the handle is located. The internal walls of the cavity 52b are smooth.

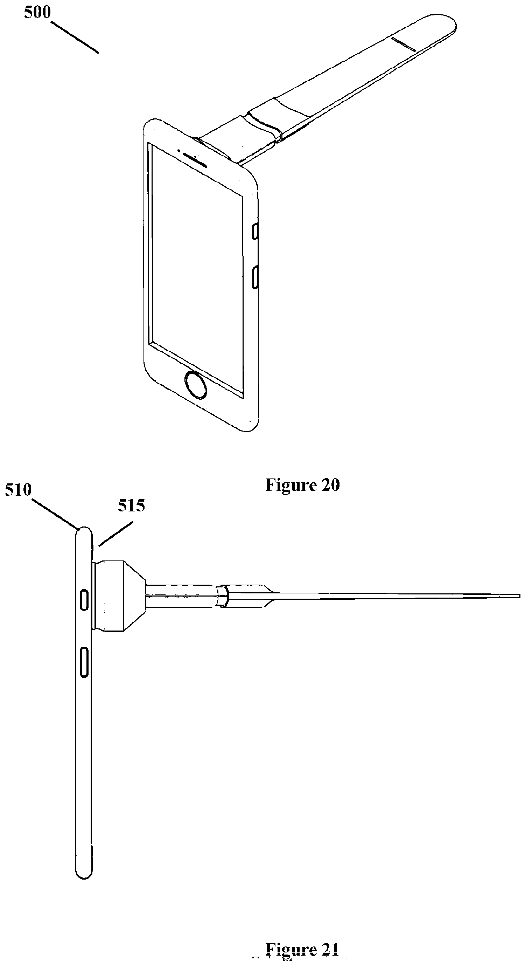

Figures 20-23 and 28 illustrate orifice inspection systems 500, 500b. The orifice inspection system 500 includes the tongue depressor 1 of Figures 14-19, including handle 10 and blade 20. The orifice inspection system 500b includes an otoscope 1b, including handle 10 and speculum 20a (or orifice inserter). A similar otoscope which could be used in the orifice inspection system 500b is shown in Figures 29-33. The proximate end portion 50a of the speculum 20a includes a cavity into which the attachment-coupling portion 13 of the handle is located.

The system 500, 500b also includes a user device 510 in the form of a smartphone, and the user device 510 includes an image capture device 515, in the form of a camera. The user device includes the application as described in Figures 1-10 or Figures 55-61. The system 500, 500b also includes a coupler 550, for releaseably coupling the handle 10 to the user device 510.

The coupler 550 is releaseably coupleable to the user device 510 by virtue of hook and loop fasteners (for example, Velcro^{™}). One strip of a hook and loop fastener is affixed to a surface of the user device 510, and another strip of a hook and loop fastener is affixed to a rear surface 552 of the coupler 550. The coupler also includes a handle engager 560, which includes a cavity. One end of the handle 10 is insertable into the handle engager 560, to thereby releaseably engage the handle 10 and the handle engager 560.

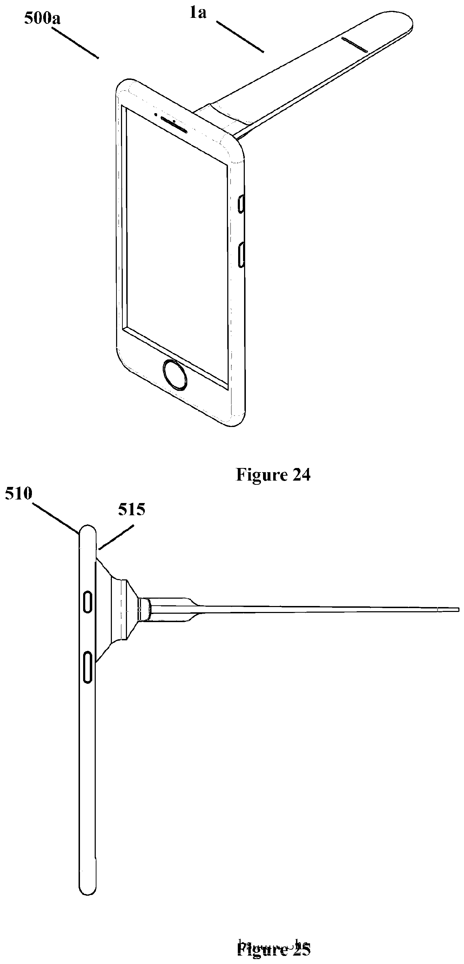

Figures 24-27 illustrate a further orifice inspection system 500a. The system 500a includes a user device 510 in the form of a smartphone, and the user device 510 includes an image capture device 515, in the form of a camera. The user device includes the application as described in Figures 1-10 or 55 to 61.

The orifice inspection system 500a also includes a tongue depressor 1a. The tongue depressor includes a handle 10a and a blade 20. The blade 20 is as described for the tongue depressor of Figures 14-19. The handle 10a includes three light sources. Light from the light source passes through the blade 20 to illuminate the oral cavity.

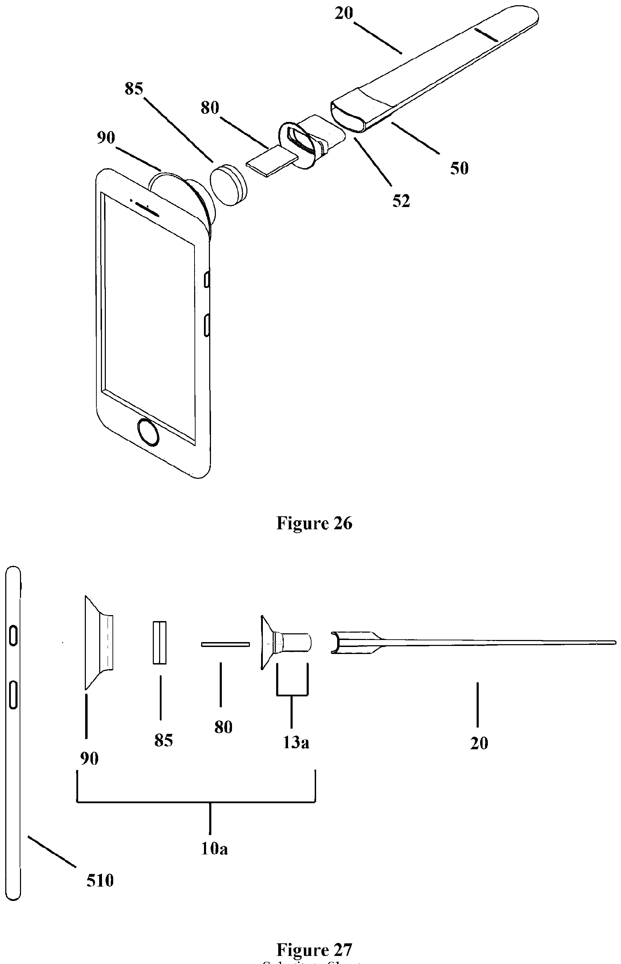

The proximate end portion 50 of the blade 20 is slideably engageable with the insert-coupling portion 13a of the handle 10a (see Figures 26-27). The proximate end portion 50 includes a cavity 52 into which the insert-coupling portion 13a of the handle is located (see Figures 26-27). The internal walls of the cavity 52 are smooth.

The handle 10a includes a circuit board 80 including a switch to connect a power supply 85 (in the form of a battery) to the light source. Coupling the blade 20 to the handle 10a actuates the switch so that light emitted from the light source is transmitted by the blade 20 to the oral cavity when the handle 10a and blade 20 are coupled together.

The insert-coupling portion 13a includes an outer wall having a flap (not shown), and the flap is positioned over the switch, so that depression of the flap by sliding the insert-coupling portion 13a into cavity 52 actuates the switch and activates the light source.

The light source is located entirely within the handle 10a. The light source is positioned adjacent to an opening in the insert-coupling portion 13a so that light from the light source passes through the opening and into the blade 20. The handle 10a includes three light sources which are positioned to extend laterally across the handle 10a. The light sources are especially light-emitting diodes (LEDs).

The power source in the handle 10 is in the form of a battery 85. The battery 85 is especially a lithium-ion battery. The battery 85 is connected to the light source and the switch via circuit board 80. The handle 10a may include a suction cap portion 90 for affixing the handle 10a to the user device 510. Separation of the suction cap portion 90 from the remainder of the handle 10a allows a user to access the battery 85, for example.

The handle 10a especially does not require sterilisation and is intended for multiple uses. The blade 20 is either sterilisable (e.g. via heat or chemical sterilisation), or is disposable. The blade 20 and the handle 10a may be sold separately.

To use the oral inspection device 1, the insert-coupling portion 13, 13a is slideably engaged into the cavity 52 of the blade 20. This actuates the switch and activates the light source 12, which transmits light into the blade 20. The blade 20 acts as an optical waveguide to direct the light to the terminal end 32 of the blade 20 for illuminating the patient's oral cavity. At least a portion of the distal end portion 30 of the blade 20 is then inserted into the patient's oral cavity to depress the tongue and illuminate the oral cavity for inspection.

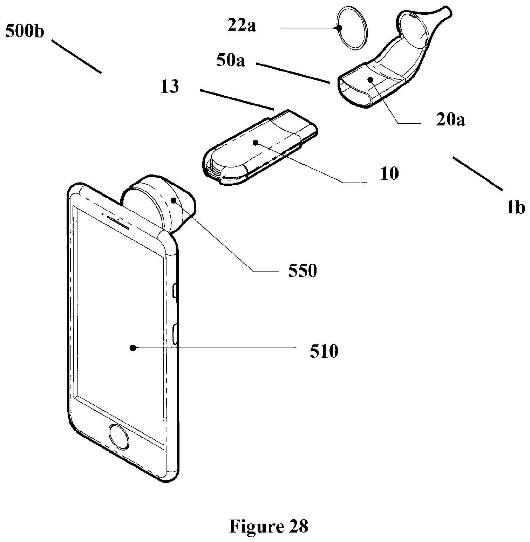

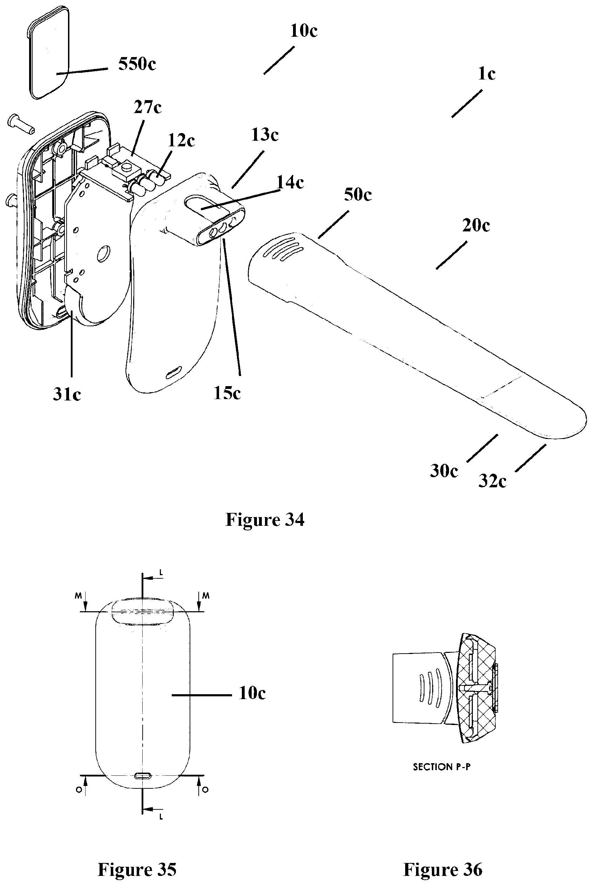

Further orifice inspection devices 1c, 1d are illustrated in Figures 34-46. While the orifice inspection devices 1c, 1d are illustrated in the form of a tongue depressor, the speculum 20a, 20b of Figures 28-33 could be used with the handle 10c, 10d of the device 1c, 1d, instead of the tongue depressor blade 20c. Tongue depressor blade 20c is identical to the tongue depressor blade 20 discussed above in Figures 14-19.

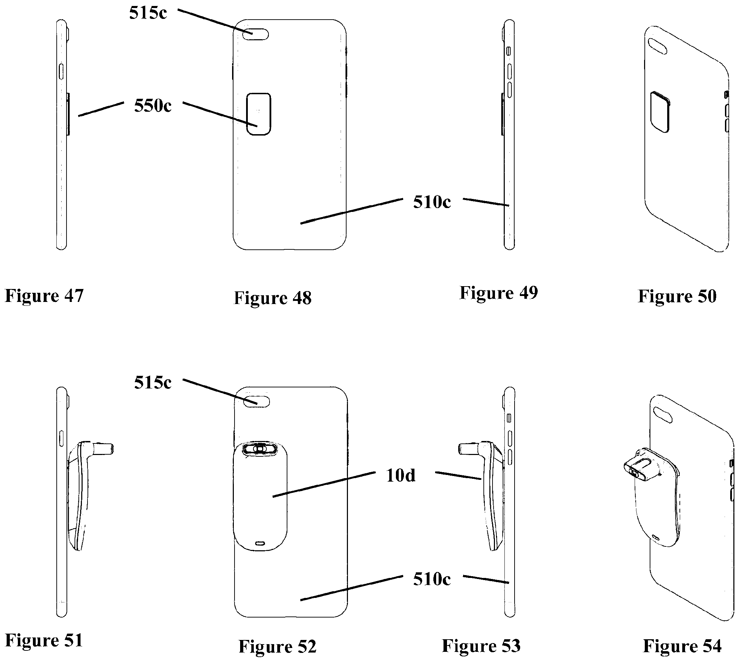

The orifice inspection devices 1c, 1d include a coupler 550c in the form of a handle engager. The handle engager 550c is planar and is slideably engageable with the handle 10c, 10d. The handle engager includes an adhesive layer for fastening the handle engager 10c, 10d with a user device such as a smart phone 510c (see Figures 47-54). The user device includes image capture device 515c. The adhesive layer is VHB Tape PX5011, manufactured by 3M.

Handle 10c includes 3 3mm LED lights 12c, and handle 10d includes one surface mount LED 12d. Light from lights 12c, 12d pass through the blade 20c to illuminate the oral cavity. The blade 20c is releaseably engageable from the handle 10c, 10d. The blade 20c acts as an optical waveguide for light emitted from the light source 12c, 12d. The blade 20c is a solid body extending from adjacent the light source 12c, 12d to its terminal end 32c. The blade 20c is adapted to emit light from the terminal end 32c. The blade 20c may be made from injection moulded plastic and may be disposable.

The blade 20c includes a distal end portion 30c having a terminal end 32c. The distal end portion 30c is substantially flat in a longitudinal and lateral direction.

At least a portion of the distal end portion 30c is intended to be inserted into the patient's oral cavity. The blade 20c also includes a proximate end portion 50c. The proximate end portion 50c of the blade 20c is slideably engageable with the insert-coupling portion 13c, 13d of the handle 10c, 10d. The proximate end portion 50c includes a cavity (not shown) into which the insert-coupling portion 13c, 13d of the handle 10c, 10d is located. The internal walls of the cavity are smooth.

The handle 10c, 10d includes a switch 27c, 27d to connect a power source 31c, 31d to the light source 12c, 12d. Coupling the blade 20c to the handle 10c, 10d actuates the switch 27c, 27d so that light emitted from the light source 12c, 12d is transmitted by the blade 20c to the oral cavity when the handle 10c, 10d and blade 20c are coupled together.

The insert-coupling portion 13c, 13d includes an outer wall having a flap 14c, 14d, and the flap 14c, 14d is positioned over the switch 27c, 27d, so that depression of the flap 14c, 14d by sliding the insert-coupling portion 13c, 13d into cavity actuates the switch 27c, 27d and activates the light source 12c, 12d.

The light source 12c, 12d is located entirely within the handle 10c, 10d. The light source is positioned adjacent to an opening 15c, 15d in the insert-coupling portion 13c, 13d so that light from the light source 12c, 12d passes through the opening 15c, 15d and into the blade 20c.

The power source 31c, 31d in the handle 10c, 10d is in the form of a lithium ion battery. The battery is connected to the light source 12c, 12d and the switch 27c, 27d via a circuit board. The handle 10c, 10d may be made from injection moulded plastic.

In use, the coupler 550c is fastened to the smart phone 510c. The handle 10c, 10d is then slideably engaged with the coupler. Advantageously, this engagement provides a rigid connection between the handle 10c, 10d and the smart phone 510c.

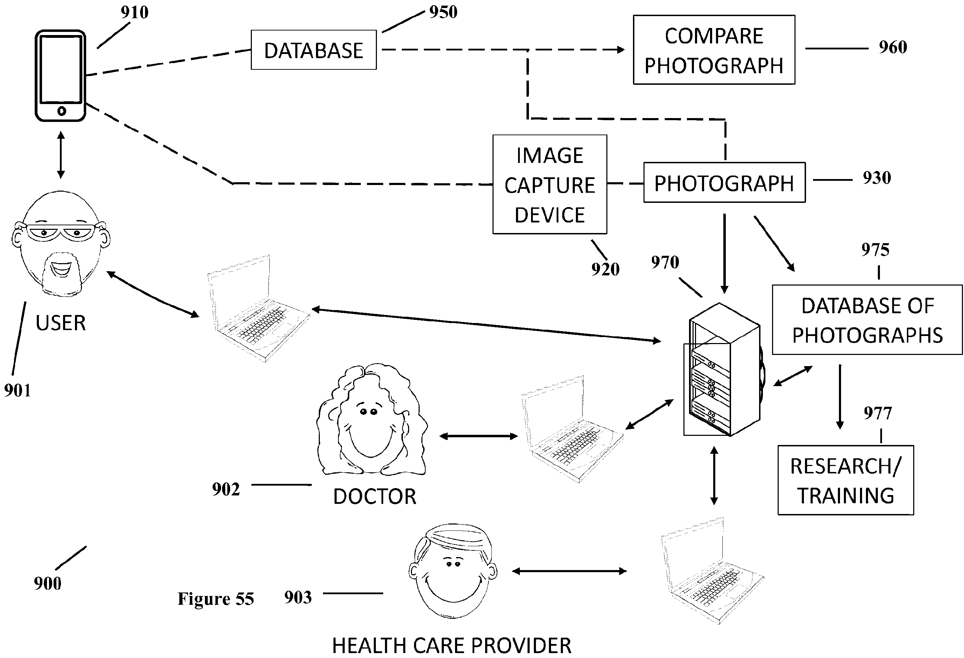

Figure 55 illustrates a further example orifice inspection system 900, according to an embodiment of the present invention. The orifice inspection system 900 may be useable domestically (for example, by a patient or a family member of the patient), or by a medical professional (for example in a doctor's surgery or hospital). The system 900 is similar to the system 100 shown in Figure 1.

As an example, a user 901 may have an application on their computer or smartphone 910. The application uses an image capture device 920 (such as a camera or webcam) to capture a photograph 930 of a patient's orifice. The image capture device may be associated with an orifice inspective device (as previously described), and the system 900 may detect the presence of the orifice inspection device and automatically adjust the image magnification of the image capture device 920 based on the location of the orifice inspection device. A database 950 of at least one photograph of an orifice having a known condition may be accessible via the graphical user interface. The graphical user interface may be used to compare the photograph 930 with images from the database at 960. For example, the graphical user interface may be configured to display the captured photograph 930 and at least one photograph of an orifice having a known condition for comparison, to assist in identifying a condition affecting the patient's orifice.

The user 901 may use a data interface on the computer or smartphone 910 in order to transmit the photograph to the patient's medical service provider. For example, the data interface may transmit the photograph 930 by sending an email to the medical service provider. The data interface may also transmit the photograph 930 by uploading the photograph onto a server 970, from which one or more of the user 901 or the medical service provider (such as a doctor 902, or other health care provider 903) may view the photograph 930. The photograph 930 may also be uploaded onto a database 975 for use in research/training 977.

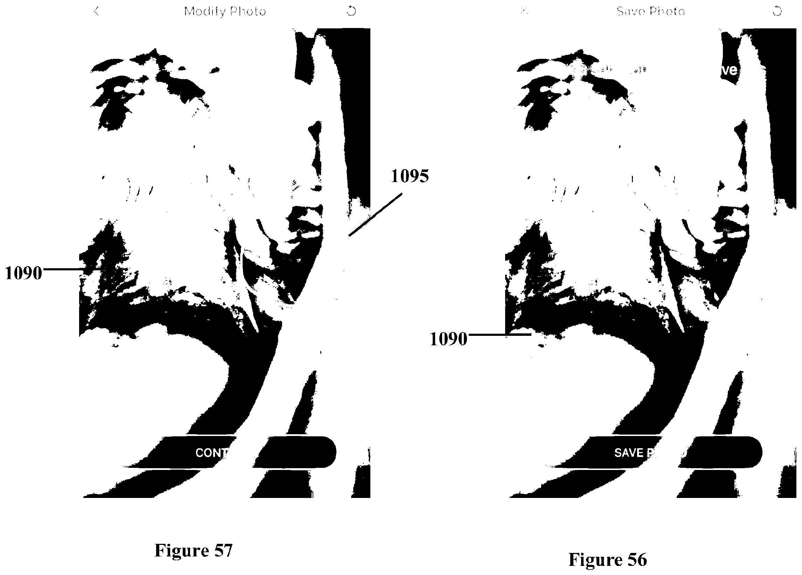

An exemplary orifice inspection system for the oral cavity will now be described with reference to Figures 2 and 56 to 60. The orifice inspection system exemplified in Figures 56 to 60 includes an application for a smartphone.

First, Figure 2 illustrates a main menu screen 200 for the application. From this screen the user can choose to take a photograph of an oral cavity 202, or browse a database showing at least one oral cavity having a known condition 204.

If the user choses to take a photograph 202, a graphical user interface is provided showing an image to be captured by an image capture device. The orifice positioning device is mounted to a smart phone (for example), and the system automatically adjusts the image magnification of the image capture device based on the location of the orifice inspection device. This is accomplished based on knowledge of the length that the orifice inspection device extends from the smart phone. When the image is in the correct position, the user can capture the photograph 1090, as shown in Figure 56.

The user then can modify the photograph 1090, as shown in Figure 57. In Figure 57 the user has highlighted a portion of the photograph 1095 for closer examination by a medical service provider.

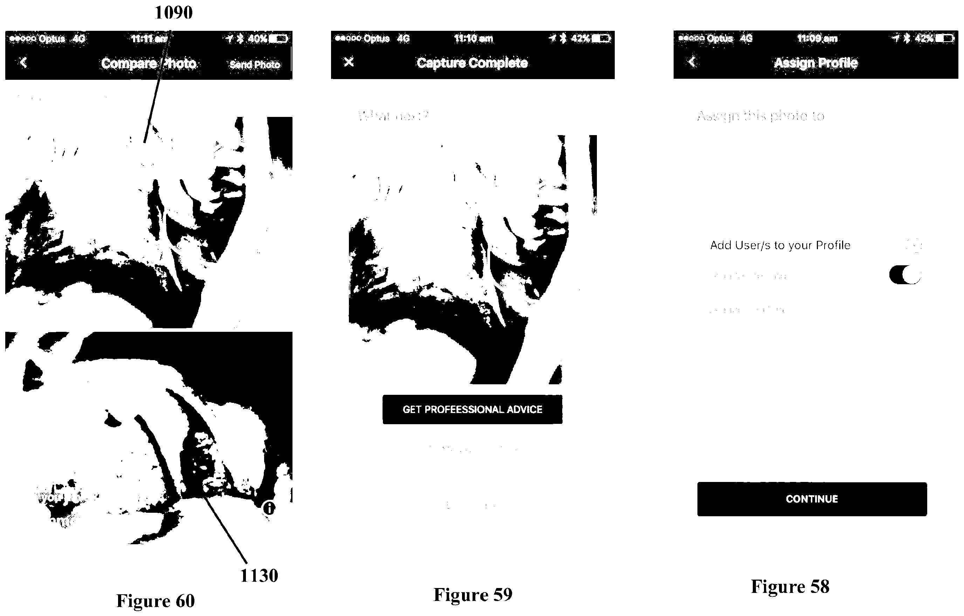

The user then can assign the photograph 1090 to their user profile, as shown in Figure 58. In this process, the user's profile may be associated with a medical service provider's profile and the photograph can be assigned to the medical service provider. The user can also seek professional advice, as shown in Figure 59.

If the user chooses to compare the photograph, the graphical user interface displays the captured photograph 1090, and at least one photograph of an oral cavity having a known condition 1130 (see Figure 60). In Figure 60, the user is able to change the photograph of the oral cavity having a known condition 1130 to compare photographs of acute tonsillitis, mononucleosis, pharyngitis, and streptococcal pharyngitis. The user may click on the image of the oral cavity having a known condition 1130 to obtain further information on the condition (as shown in Figure 6).

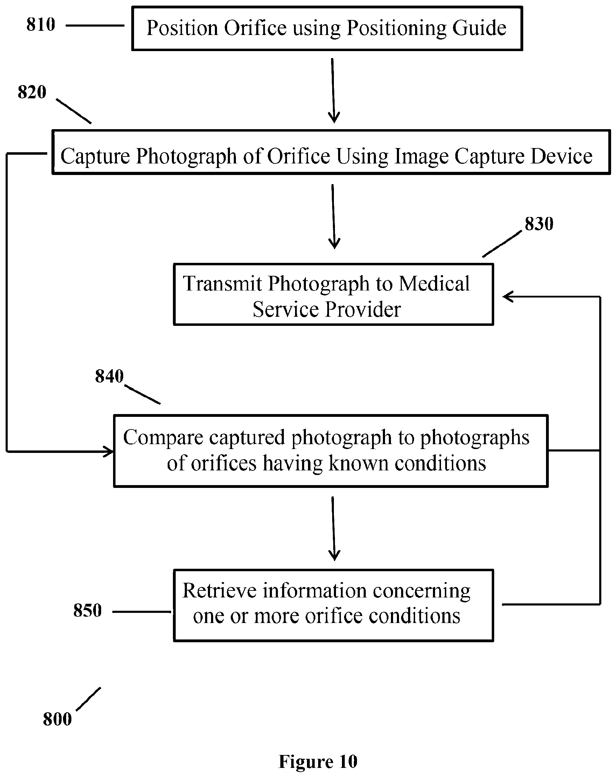

Figure 10 illustrates an orifice inspection method 800. The method may include the step of coupling an orifice inserter (such as blade 20) to a handle 10, to thereby form an orifice inspection device 1. A light source 12 in the orifice inspection device may be activated by coupling the orifice inserter to the handle 10. A coupler may be releaseably engaged with the handle 10, and the coupler may also be releaseably engaged with a smartphone.

Using a graphical user interface, the patient's orifice is positioned using the orifice positioning guide 810. At step 820, after the patient's orifice has been positioned, a photograph of the orifice is captured using the image capture device. The user may then choose to transmit the photograph to a medical service provider 830, or to compare the captured photograph to photographs of orifices having known conditions 840. The user may retrieve information concerning the known conditions at step 850. Following steps 840 or 850, the user may also choose to transmit the photograph to a medical service provider 830.

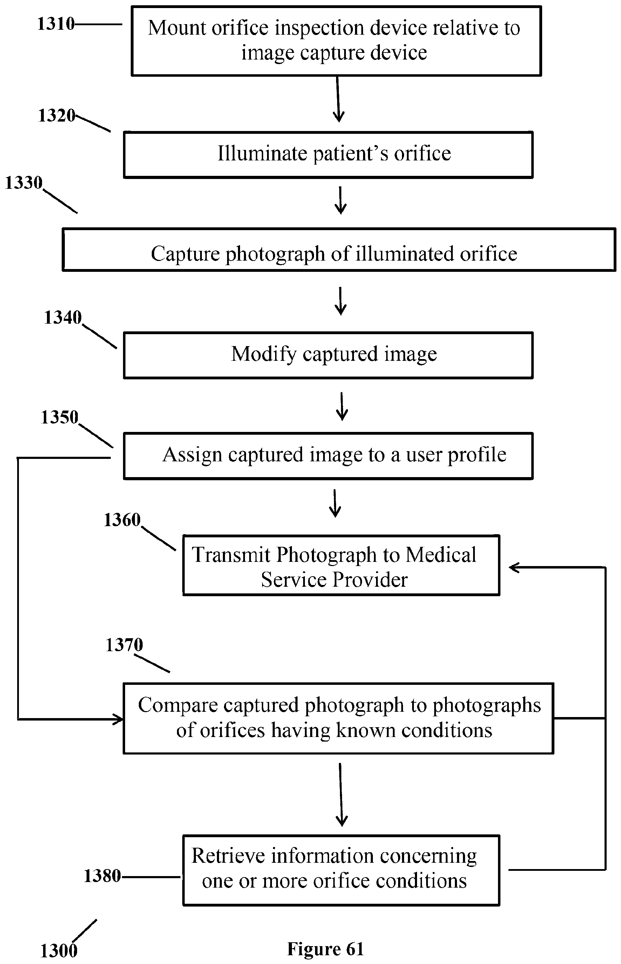

Figure 61 illustrates a further orifice inspection method 1300. The method may include the step of mounting an orifice inspection device relative to an image capture device 1310. For example, a coupler may be fastened to a user device such as a smart phone, and a handle 10-10d may be releasably engaged with the coupler. The method may include the step of coupling an orifice inserter (such as blade 20-20c) to a handle 10-10d, to thereby form an orifice inspection device 1-1d. A light source 12-12d in the orifice inspection device may be activated by coupling the orifice inserter to the handle 10-10d.

The patient's orifice is then illuminated 1320, and using a graphical user interface an image is captured 1330. The user has the option of modifying the captured image 1340, for example by highlighting a portion. The photograph is then assigned to a user profile 1350, and then the photograph can be transmitted to a medical service provider 1360 or the photograph can be compared with photographs of orifices having known conditions 1370. Information concerning one or more of the orifice conditions may also be retrieved 1380.

Reference throughout this specification to 'one embodiment' or 'an embodiment' means that a particular feature, structure, or characteristic described in connection with the embodiment is included in at least one embodiment of the present invention. Thus, the appearance of the phrases 'in one embodiment' or 'in an embodiment' in various places throughout this specification are not necessarily all referring to the same embodiment. Furthermore, the particular features, structures, or characteristics may be combined in any suitable manner in one or more combinations.

In compliance with the statute, the invention has been described in language more or less specific to structural or methodical features. It is to be understood that the invention is not limited to specific features shown or described since the means herein described includes preferred forms of putting the invention into effect. The invention is, therefore, claimed in any of its forms or modifications within the proper scope of the appended claims appropriately interpreted by those skilled in the art.

## Claims

1. An orifice inspection system (100), including:
- An orifice inspection device (1) for illuminating a patient's orifice, the orifice inspection device including a handle (10c) with a light source (12c), a power source (31c) and an orifice-insertable disposable light transmissive structure (20c) formed of a light transmissive material releasably connectable to the handle, the handle including a handle body and a coupling portion (13c) extending therefrom, the light source being located within the coupling portion of the handle and the coupling portion including a switch (27c) in communication with the light source and the power source, and the disposable light transmissive structure includes a cavity to receive the coupling portion of the handle and activate the switch such that the light source is directed therealong;
- A smart phone (510c) including a screen and an image capture device (515) for capturing a photograph of the patient's orifice as illuminated by the orifice inspection device; and
- A coupler (500c) arranged to attach with the smart phone so as to releasably couple the handle body of the orifice inspection device to the smart phone thereby positioning the disposable light transmissive structure relative to the image capture device;
- wherein the orifice inspection device is operable in a first condition disconnected from the smart phone in which a user may clasp the handle body to illuminate the patient's orifice, and a second condition in which the handle body is coupled to the smart phone so as to be immediately adjacent the smart phone with the disposable light transmissive structure laterally spaced apart from the image capture device so that the image capture device is unobstructed thereby allowing the user to capture the photograph of the patient's orifice surrounding the disposable light transmissive structure whilst holding the smart phone.

2. The orifice inspection system according to claim 1, wherein the handle body is slidable onto the coupler.

3. The orifice inspection system according to claim 1, wherein the coupler is positioned on a rear surface of the smart phone and spaced apart from a lens of the image capture device.

4. The orifice inspection system according to claim 1, wherein the coupler and the handle are arranged such that the disposable light transmissive structure is positioned non-obstructively adjacent to and extending in a direction away from a lens of the image capture device.

5. The orifice inspection system according to claim 1, wherein the disposable light transmissive structure is removable from the handle irrespective of whether or not the handle is coupled to the coupler.

6. The orifice inspection system of claim 1, wherein the orifice inspection system further includes a graphical user interface presented on a screen of the smart phone including an orifice positioning guide for positioning a patient's orifice in the photograph captured by the image capturing device.

7. The orifice inspection system of claim 1, wherein the orifice inspection system further includes a data interface for transmitting the photograph to the patient's medical service provider.

8. The orifice inspection system of claim 1, wherein the orifice inspection system further includes a graphical user interface configured to display the captured photograph and at least one photograph of an orifice having a known condition for comparison, to assist in identifying a condition affecting the patient's orifice.

9. The orifice inspection system of claim 1, wherein the orifice inspection device is for inspecting an oral cavity, an outer ear or a nostril.

10. The orifice inspection system of claim 1, wherein the coupler is planar.

11. The orifice inspection system of claim 1, wherein the light source includes a plurality of LED lights extending across the coupling portion of the handle.

## Patentansprüche

1. Körperöffnungs-Inspektionssystem (100), umfassend:
- Körperöffnungs-Inspektionsvorrichtung (1) für die Beleuchtung der Körperöffnung eines Patienten, die Körperöffnungs-Inspektionsvorrichtung umfasst einen Griff (10c) mit einer Lichtquelle (12c), einer Stromquelle (31c) und einer in die Körperöffnung einführbaren lichtdurchlässigen Einwegstruktur (20c), die aus einem lichtdurchlässigen Material geformt ist, das abnehmbar mit dem Griff verbunden werden kann, der Griff umfasst einen Griffkörper und einen Kopplungsabschnitt (13c), der sich daraus erstreckt, die Lichtquelle ist in dem Kopplungsabschnitt des Griffs untergebracht und der Kopplungsabschnitt beinhaltet einen Schalter (27c) in Verbindung mit der Lichtquelle und der Stromquelle und die lichtdurchlässige Einwegstruktur beinhaltet eine Aussparung, um den Kopplungsabschnitt des Griffs aufzunehmen und den Schalter zu aktivieren, so dass die Lichtquelle dort entlang geleitet wird;
- Smartphone (510c) mit einem Display und einer Bildaufnahmevorrichtung (515) für das Aufnehmen einer Fotografie der Körperöffnung des Patienten, die von der Körperöffnungs-Inspektionsvorrichtung beleuchtet wird; und
- Verbindungsstück (500c), das so angeordnet ist, dass es am Smartphone befestigt werden kann, um den Griffkörper der Körperöffnungs-Inspektionsvorrichtung abnehmbar mit dem Smartphone zu koppeln und dadurch die lichtdurchlässige Einwegstruktur im Verhältnis zur Bildaufnahmevorrichtung zu positionieren;
wobei die Körperöffnungs-Inspektionsvorrichtung in einem ersten Zustand getrennt vom Smartphone betriebsfähig ist, in dem ein Benutzer den Griffkörper greifen kann, um die Körperöffnung des Patienten zu beleuchten, und in einem zweiten Zustand, in dem der Griffkörper mit dem Smartphone gekoppelt wird, damit er direkt am Smartphone mit der lichtdurchlässigen Einwegstruktur seitlich mit Abstand zur Bildaufnahmevorrichtung anliegt, so dass die Bildaufnahmevorrichtung nicht verdeckt wird und dadurch der Benutzer die Fotografie der Körperöffnung des Patienten um die lichtdurchlässige Einwegstruktur herum aufnehmen kann, während er das Smartphone in der Hand hält.

2. Körperöffnungs-Inspektionssystem nach Anspruch 1, wobei der Griffkörper auf das Verbindungsstück verschiebbar ist.

3. Körperöffnungs-Inspektionssystem nach Anspruch 1, wobei das Verbindungsstück an einer Rückseite des Smartphones mit Abstand zu einer Linse der Bildaufnahmevorrichtung positioniert ist.

4. Körperöffnungs-Inspektionssystem nach Anspruch 1, wobei das Verbindungsstück und der Griff so angeordnet sind, dass die lichtdurchlässige Einwegstruktur so positioniert ist, dass sie eine Linse der Bildaufnahmevorrichtung nicht versperrt und sich in eine Richtung weg davon erstreckt.

5. Körperöffnungs-Inspektionssystem nach Anspruch 1, wobei die lichtdurchlässige Einwegstruktur vom Griff abnehmbar ist, unabhängig davon, ob der Griff mit dem Verbindungsstück gekoppelt ist oder nicht.

6. Körperöffnungs-Inspektionssystem nach Anspruch 1, wobei das Körperöffnungs-Inspektionssystem ferner eine grafische Benutzeroberfläche umfasst, die auf einem Display des Smartphones dargestellt wird, einschließlich einer Körperöffnungs-Positionierungsführung für die Positionierung einer Körperöffnung eines Patienten auf der Fotografie, die von der Bildaufnahmevorrichtung aufgenommen wird.

7. Körperöffnungs-Inspektionssystem nach Anspruch 1, wobei das Körperöffnungs-Inspektionssystem ferner eine Datenschnittstelle umfasst, die die Fotografie an den medizinischen Service-Provider des Patienten übermittelt.

8. Körperöffnungs-Inspektionssystem nach Anspruch 1, wobei das Körperöffnungs-Inspektionssystem ferner eine grafische Benutzeroberfläche umfasst, die konfiguriert ist, die aufgenommene Fotografie und mindestens eine Fotografie einer Körperöffnung mit einer bekannten Krankheit zum Vergleich zu zeigen, um bei der Identifizierung einer Krankheit zu helfen, die die Körperöffnung des Patienten betrifft.

9. Körperöffnungs-Inspektionssystem nach Anspruch 1, wobei die Körperöffnungs-Inspektionsvorrichtung der Inspektion einer Mundhöhle, einem Außenohr oder einem Nasenloch dient.

10. Körperöffnungs-Inspektionssystem nach Anspruch 1, wobei das Verbindungsstück eben ist.

11. Körperöffnungs-Inspektionssystem nach Anspruch 1, wobei die Lichtquelle eine Vielzahl von LED-Leuchten umfasst, die sich über den Verbindungsabschnitt des Griffs erstrecken.

## Revendications

1. Système d'inspection d'orifice (100), comportant :
- un dispositif d'inspection d'orifice (1) destiné à éclairer l'orifice d'un patient, le dispositif d'inspection d'orifice comportant une poignée (10c) dotée d'une source de lumière (12c), une source d'alimentation (31c) et une structure jetable transmettant la lumière insérable par orifice (20c) constituée d'un matériau de transmission de lumière pouvant être raccordé de manière amovible à la poignée, la poignée comportant un corps de poignée et une partie d'accouplement (13c) s'étendant à partir de celle-ci, la source de lumière étant située à l'intérieur de la partie d'accouplement de la poignée et la partie d'accouplement comportant un commutateur (27c) en communication avec la source de lumière et la source d'alimentation et la structure jetable transmettant la lumière comporte une cavité pour recevoir la partie d'accouplement de la poignée et activer le commutateur de sorte que la source de lumière soit dirigée le long de celle-ci ;
- un téléphone intelligent (510c) comportant un écran et un dispositif de capture d'image (515) destiné à capturer une photographie de l'orifice du patient tel qu'éclairé par le dispositif d'inspection d'orifice ; et
- un accouplement (500c) agencé pour se fixer au téléphone intelligent de façon à accoupler de manière amovible le corps de poignée du dispositif d'inspection d'orifice au téléphone intelligent, positionnant ainsi la structure jetable transmettant la lumière par rapport au dispositif de capture d'image ;
dans lequel le dispositif d'inspection d'orifice est utilisable dans un premier état non raccordé au téléphone intelligent dans lequel un utilisateur peut saisir le corps de poignée pour éclairer l'orifice du patient et un second état dans lequel le corps de poignée est accouplé au téléphone intelligent de manière à être immédiatement adjacent au téléphone intelligent avec la structure jetable transmettant la lumière espacée latéralement du dispositif de capture d'image de manière à ce que le dispositif de capture d'image ne soit pas obstrué, ce qui permet à l'utilisateur de capturer la photographie de l'orifice du patient entourant la structure jetable transmettant la lumière tout en tenant le téléphone intelligent.

2. Système d'inspection d'orifices selon la revendication 1, dans lequel le corps de poignée est coulissant sur l'accouplement.

3. Système d'inspection d'orifice selon la revendication 1, dans lequel l'accouplement est positionné sur une surface arrière du téléphone intelligent et espacé d'une lentille du dispositif de capture d'image.

4. Système d'inspection d'orifice selon la revendication 1, dans lequel l'accouplement et la poignée sont agencés de sorte que la structure jetable transmettant la lumière est positionnée de manière non obstructive adjacente à une direction éloignée d'une lentille du dispositif de capture d'image et s'étendant dans celle-ci.

5. Système d'inspection d'orifices selon la revendication 1, dans lequel la structure jetable transmettant la lumière peut être retirée de la poignée, que la poignée soit accouplée ou non à l'accouplement.

6. Système d'inspection d'orifice selon la revendication 1, dans lequel le système d'inspection d'orifice comporte en outre une interface utilisateur graphique présentée sur un écran du téléphone intelligent comportant un guide destiné au positionnement de l'orifice pour positionner l'orifice d'un patient dans la photographie capturée par le dispositif de capture d'image.

7. Système d'inspection d'orifice selon la revendication 1, dans lequel le système d'inspection d'orifice comporte en outre une interface de données destinée à transmettre la photographie au prestataire de services médicaux du patient.

8. Système d'inspection d'orifice selon la revendication 1, dans lequel le système d'inspection d'orifice comporte en outre une interface utilisateur graphique configurée pour afficher la photographie capturée et au moins une photographie d'un orifice présentant un état connu pour comparaison, afin d'aider à identifier un état de santé affectant l'orifice du patient.

9. Système d'inspection d'orifice selon la revendication 1, dans lequel le dispositif d'inspection d'orifice est destiné à inspecter une cavité orale, une oreille externe ou une narine.

10. Système d'inspection d'orifice selon la revendication 1 dans lequel l'accouplement est plat.

11. Système d'inspection d'orifice selon la revendication 1, dans lequel la source de lumière comporte une pluralité de voyants à DEL s'étendant sur l'ensemble de la partie d'accouplement de la poignée.
